# EUROPEAN PATENT APPLICATION

(11) **EP 3 590 342 A1**
(43) Date of publication of application: **08.01.2020**
(21) Application number: 18760589.4
(22) Date of filing: 02.03.2018
(51) Int. Cl.: A01N 47/38, A01P 21/00, C07D 249/04, C07D 401/06, C07D 401/12, C07D 403/06, C07D 413/12, C07D 417/12

(54) **STRIGOLACTONE RECEPTOR INHIBITOR, AGRICULTURAL COMPOSITION AND USE THEREOF, STRIGA SEED GERMINATION INHIBITOR, AND TRIAZOLE UREA COMPOUND**

(30) Priority: 03.03.2017 JP 2017040881
(71) Applicant: The University of Tokyo, Bunkyo-ku Tokyo 113-8654 (JP)
(72) Inventor: ASAMI Tadao, Tokyo 113-8654 (JP); NAKAMURA Hidemitsu, Tokyo 113-8654 (JP); KIKUZATO Ko, Tokyo 113-8654 (JP); HU Wenqian, Tokyo 113-8654 (JP)
(74) Representative: Schiener, Jens
(86) International application number: PCT/JP2018/008121
(87) International publication number: WO 2018/159835

(57) **Abstract**

The present invention provides a strigolactone receptor inhibitor for plants, the strigolactone receptor inhibitor containing at least one kind of triazole urea compounds represented by the following formula (I) as an active ingredient; an agricultural composition; a method for using the agricultural composition as, for example, a branching enhancer for plants; a germination inhibitor for *Striga* seeds, the germination inhibitor containing the triazole urea compound; and a novel triazole urea compound that is useful as a strigolactone receptor inhibitor for plants. In the formula, A represents a group represented by (A-1) or (A-2). R¹ and R² represent a hydrogen atom and so forth. R³ and R⁴ represent a hydrogen atom, an alkyl group having 1 to 20 carbon atoms, a cycloalkyl group having 3 to 20 carbon atoms, or an aryl group having 6 to 20 carbon atoms and so forth. In addition, R³ and R⁴ may form a ring together.

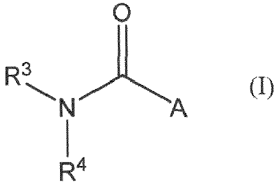

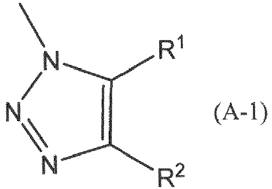

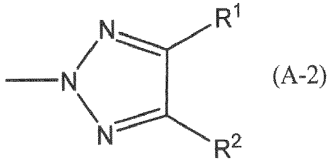

## Description

### TECHNICAL FIELD

The present invention relates to a strigolactone receptor inhibitor for plants containing a triazole urea compound as an active ingredient, an agricultural composition containing the triazole urea compound, a method for using the agricultural composition as a branching enhancer and the like for plants, a germination inhibitor for *Striga* seeds containing the triazole urea compound, and a novel triazole urea compound.

### BACKGROUND ART

A strigolactone is known as a seed germination accelerating substance for *Striga,* a root parasitic weed, referred to as a witchweed that causes a serious damage on farm products in Africa. In recent years, it has also been known as a plant hormone that has multiple activities, such as a branching inhibitory effect. Therefore, clarification of a structure of a receptor of the strigolactone and its signal transmission mechanism has been an important object in terms of growth control and the like of plants (Non-Patent Document 1).

In relation to the present invention, Non-Patent Document 2 describes that triazole urea compounds having partial structures represented by the following formulae (α) and (β) are inhibitory substances of serine hydrases of animals.

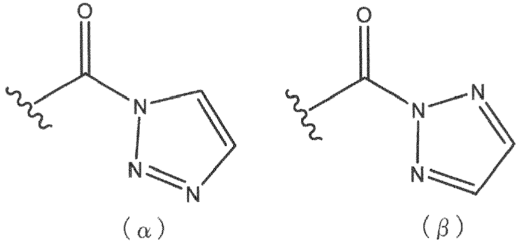

However, this document does not describe that these compounds are inhibitory substances of the strigolactone receptor of plants. While the strigolactone receptor of plants has been known as a protein having a serine hydrase activity, it has not been clarified if the serine hydrase activity is necessary for a strigolactone reception signal transmission, and it is not clear from the conventional knowledge whether the serine hydrase activity inhibitory substance inhibits the strigolactone reception or not.

Non-Patent Document 1: Chemistry and Biology, Vol. 53, No. 3, 2015
Non-Patent Document 2: Nature Chemical Biology, Vol. 7, July 2011

### DISCLOSURE OF THE INVENTION

### PROBLEMS TO BE SOLVED BY THE INVENTION

Objectives of the present invention are to provide a strigolactone receptor inhibitor for plants containing a triazole urea compound having a specific structure as an active ingredient, an agricultural composition containing the triazole urea compound, a method for using the agricultural composition as a branching enhancer and the like for plants, a germination inhibitor for *Striga* seeds containing the triazole urea compound, and a novel triazole urea compound effective as a strigolactone receptor inhibitor and the like for plants.

### SOLUTIONS TO THE PROBLEMS

While studying a structure of a receptor of a strigolactone and its signal transmission mechanism, the inventors have found that: a triazole urea compound represented by the later-described formula (I) exhibits a strong strigolactone antagonist activity and has a germination inhibitory effect for *Striga* seeds; a rice d17 mutant (rice strigolactone biosynthesis mutant) exhibits a remarkable tillering recovery when the triazole urea compound represented by the formula (I) is given to the rice d17 mutant; and tillering is accelerated when a wild-type rice (variety name: Nipponbare) is grown by using a hydroponic solution containing the triazole urea compound represented by the formula (I), and thus, the inventors have completed the present invention.

Thus, the present invention provides strigolactone receptor inhibitors for plants in the following (1) and (2), agricultural compositions in (3) and (4), using methods in (5) and (6), a germination inhibitor for *Striga* seeds in (7), and a triazole urea compound in (8).
(1) A strigolactone receptor inhibitor for plants, the strigolactone receptor inhibitor containing at least one kind of triazole urea compounds represented by the following formula (I) as an active ingredient. 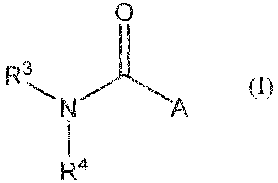
   [In the formula, A represents a group represented by the following (A-1) or (A-2). 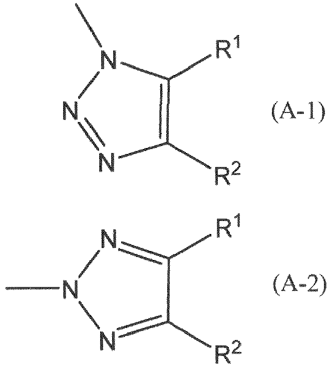
   (In the formula, each of R¹ and R² independently represents a hydrogen atom, an alkyl group having 1 to 20 carbon atoms that may have a substituent, or an aryl group having 6 to 20 carbon atoms that may have a substituent.)
   Each of R³ and R⁴ independently represents a hydrogen atom, an alkyl group having 1 to 20 carbon atoms that may have a substituent, a cycloalkyl group having 3 to 20 carbon atoms that may have a substituent, or an aryl group having 6 to 20 carbon atoms that may have a substituent (where a case where both R³ and R⁴ are hydrogen atoms is excluded). R³ and R⁴ may form a ring together, and the ring may have a substituent at any position.]
(2) The strigolactone receptor inhibitor for plants according to (1), wherein a group represented by -N(R³)(R⁴) in the formula (I) is any one of groups represented by the following (B-1) to (B-7). 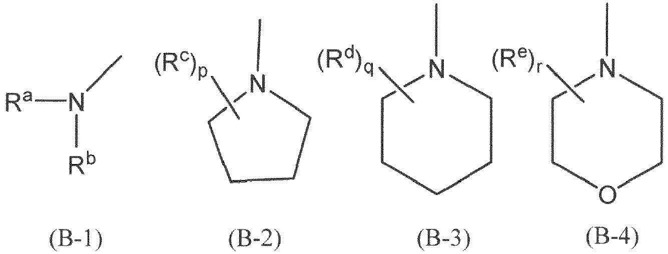 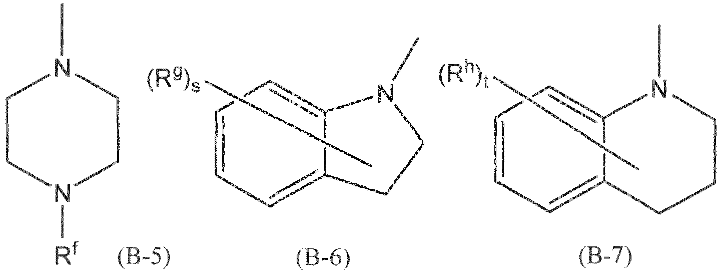
   (In the formula, each of R^{a} and R^{b} independently represents an alkyl group having 1 to 20 carbon atoms that may have a substituent or an aryl group having 6 to 20 carbon atoms that may have a substituent.
   Each of R^{c}, R^{d}, R^{e}, R^{g}, and R^{h} independently represents a nitro group, a cyano group, a halogen atom, an alkyl group having 1 to 6 carbon atoms, a haloalkyl group having 1 to 6 carbon atoms, a cycloalkyl group having 3 to 20 carbon atoms, an alkoxy group having 1 to 6 carbon atoms, an amino group, a mono-substituted amino group, a disubstituted amino group, an alkylthio group having 1 to 6 carbon atoms, an alkylsulfenyl group having 1 to 6 carbon atoms, an alkylsulfonyl group having 1 to 6 carbon atoms, an alkoxycarbonyl group having 2 to 10 carbon atoms, an alkylcarbonyl group having 2 to 10 carbon atoms, or an aryl group having 6 to 20 carbon atoms that may have a substituent.
   R^{f} represents a hydrogen atom, an alkyl group having 1 to 20 carbon atoms that may have a substituent, a cycloalkyl group having 3 to 20 carbon atoms that may have a substituent, an aryl group having 6 to 20 carbon atoms that may have a substituent, a heterocyclic group that may have a substituent, or an acyl group having 2 to 20 carbon atoms.
   Each of *p, q, r, s,* and *t* independently represents an integer of 0 to 4.)
   A bond "-" (bond extending from carbon atom constituting ring. The same applies to the following) in the above-described formula (B-2) represents that the substituent R^{c} may be substituted at any bondable position of a pyrrolidine ring. A bond "-" in the above-described formula (B-3) represents that the substituent R^{d} may be substituted at any bondable position of a piperidine ring. A bond "-" in the above-described formula (B-4) represents that the substituent R^{e} may be substituted at any bondable position of a morpholine ring. A bond "-" in (B-6) represents that the substituent R^{g} may be substituted at any bondable position of an indoline ring. A bond "-" in (B-7) represents that the substituent R^{h} may be substituted at any bondable position of a 1, 2, 3, 4-tetrahydroquinoline ring. The same applies to the following.
(3) An agricultural composition that contains at least one kind of triazole urea compounds represented by the following formula (I) and an agriculturally acceptable formulation adjuvant. 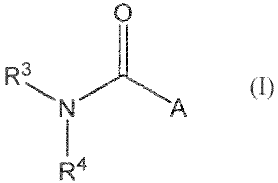
   [In the formula, A represents a group represented by the following (A-1) or (A-2). 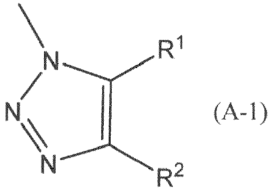 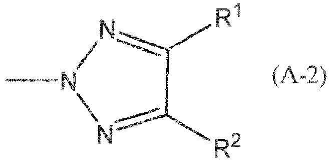
   (In the formula, each of R¹ and R² independently represents a hydrogen atom, an alkyl group having 1 to 20 carbon atoms that may have a substituent, or an aryl group having 6 to 20 carbon atoms that may have a substituent.)
   Each of R³ and R⁴ independently represents a hydrogen atom, an alkyl group having 1 to 20 carbon atoms that may have a substituent, a cycloalkyl group having 3 to 20 carbon atoms that may have a substituent, or an aryl group having 6 to 20 carbon atoms that may have a substituent (where a case where both R³ and R⁴ are hydrogen atoms is excluded). R³ and R⁴ may form a ring together, and the ring may have a substituent at any position.]
(4) The agricultural composition according to (3), wherein a group represented by -N(R³)(R⁴) in the formula (I) is any one of groups represented by the following (B-1) to (B-7). 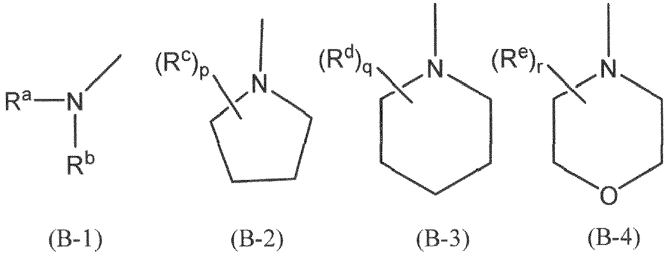 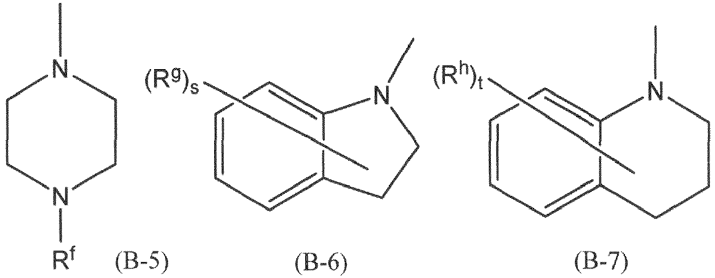
   (In the formula, each of R^{a} and R^{b} independently represents an alkyl group having 1 to 20 carbon atoms that may have a substituent or an aryl group having 6 to 20 carbon atoms that may have a substituent.
   Each of R^{c}, R^{d}, R^{e}, R^{g}, and R^{h} independently represents a nitro group, a cyano group, a halogen atom, an alkyl group having 1 to 6 carbon atoms, a haloalkyl group having 1 to 6 carbon atoms, a cycloalkyl group having 3 to 20 carbon atoms, an alkoxy group having 1 to 6 carbon atoms, an amino group, a mono-substituted amino group, a disubstituted amino group, an alkylthio group having 1 to 6 carbon atoms, an alkylsulfenyl group having 1 to 6 carbon atoms, an alkylsulfonyl group having 1 to 6 carbon atoms, an alkoxycarbonyl group having 2 to 10 carbon atoms, an alkylcarbonyl group having 2 to 10 carbon atoms, or an aryl group that may have a substituent. R^{f} represents a hydrogen atom, an alkyl group having 1 to 20 carbon atoms that may have a substituent, a cycloalkyl group having 3 to 20 carbon atoms that may have a substituent, an aryl group having 6 to 20 carbon atoms that may have a substituent, a heterocyclic group that may have a substituent, or an acyl group having 2 to 20 carbon atoms.
   Each of *p*, *q, r, s,* and *t* independently represents an integer of 0 to 4.)
(5) A method for using the strigolactone receptor inhibitor according to (1) or (2), or the agricultural composition according to (4) as a branching enhancer for plants.
(6) A method for using the strigolactone receptor inhibitor according to (1) or (2), or the agricultural composition according to (4) as a tillering accelerator for gramineous plants.
(7) A germination inhibitor for *Striga* seeds, the germination inhibitor containing at least one kind of triazole urea compounds represented by the formula (I) as an active ingredient.
(8) A triazole urea compound represented by the following formula (I-1).

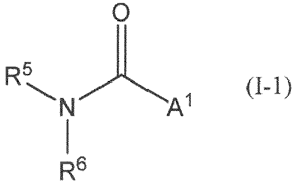

[In the formula, A¹ represents a group represented by the following (A¹-1) or (A¹-2).

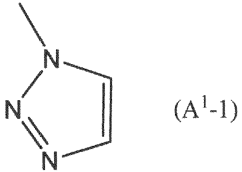

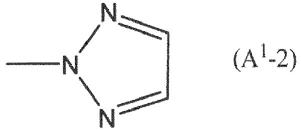

A group represented by -N(R⁷)(R⁸) represents a group represented by the following (B¹-5), (B¹-6), or (B¹-7).

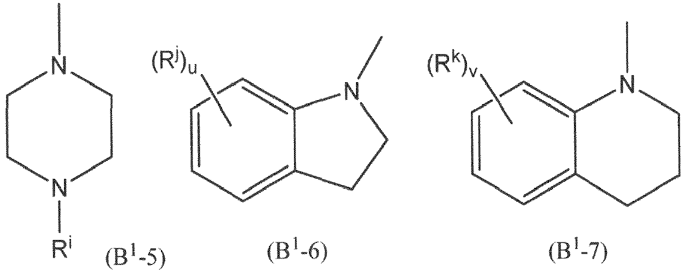

(In the formula, Rⁱ represents a hydrogen atom, an alkyl group having 1 to 20 carbon atoms that may have a substituent, a cycloalkyl group having 3 to 20 carbon atoms that may have a substituent, an aryl group having 6 to 20 carbon atoms that may have a substituent, or a heterocyclic group that may have a substituent. Each of R^{j} and R^{k} independently represents a nitro group, a cyano group, a halogen atom, an alkyl group having 1 to 6 carbon atoms, a haloalkyl group having 1 to 6 carbon atoms, a cycloalkyl group having 3 to 20 carbon atoms, an alkoxy group having 1 to 6 carbon atoms, an amino group, a mono-substituted amino group, a disubstituted amino group, an alkylthio group having 1 to 6 carbon atoms, an alkylsulfenyl group having 1 to 6 carbon atoms, an alkylsulfonyl group having 1 to 6 carbon atoms, an alkoxycarbonyl group having 2 to 10 carbon atoms, an alkylcarbonyl group having 2 to 10 carbon atoms, or an aryl group having 6 to 20 carbon atoms that may have a substituent.
Each of *u* and *v* independently represents an integer of 0 to 4.)

A bond "-" in the above-described formula (B¹-6) represents that the substituent R^{j} may be substituted at any bondable position of an indoline ring. A bond "-" in (B¹-7) represents that the substituent R^{k} may be substituted at any bondable position of a 1,2,3,4-tetrahydroquinoline ring. The same applies to the following.

### BRIEF DESCRIPTION OF THE DRAWINGS

FIG. 1 is a schematic diagram illustrating an effect of a strigolactone receptor inhibitor of the present invention on a branching trait of a rice plant.
FIG. 2 is evaluation test results of the strigolactone receptor inhibitor using a rice d17 mutant strain.
FIG. 3 is evaluation test results of the strigolactone receptor inhibitor using the rice d17 mutant strain.
FIG. 4 is graphs indicating results that have measured lengths of a first tillering and a second tillering of a rice plant that is a wild-type rice (variety name: Nipponbare) of seven days from the germination has been grown in a hydroponic solution containing 10 µM of a compound 25.
FIG. 5(a) is a photograph of a wild-type rice after a lapse of two weeks since the germination, and FIG. 5(b) is a photograph of a rice plant that has been grown in the hydroponic solution containing 10 µM of the compound 25 since seven days after the germination.

### DESCRIPTION OF PREFERRED EMBODIMENTS

The following describes the present invention in details.

It should be noted that carbon atoms of an alkyl group having 1 to 20 carbon atoms that may have a substituent, a cycloalkyl group having 3 to 20 carbon atoms that may have a substituent, and an aryl group having 6 to 20 carbon atoms that may have a substituent do not include carbon atoms of the substituents in the description.

Note that "may have a substituent" means "unsubstituted" or "have a substituent."

### 1) Strigolactone Receptor Inhibitor for Plants

A strigolactone receptor inhibitor for plants of the present invention contains at least one kind of triazole urea compounds represented by the formula (I) as an active ingredient.

### [Triazole Urea Compound Represented by Formula (I)]

In the formula (I), A represents a group represented by the formula (A-1) or (A-2).

In the formulae (A-1) and (A-2), each of R¹ and R² independently represents a hydrogen atom, an alkyl group having 1 to 20 carbon atoms that may have a substituent, or an aryl group having 6 to 20 carbon atoms that may have a substituent.)

The alkyl group having 1 to 20 carbon atoms of the alkyl group having 1 to 20 carbon atoms that may have a substituent of R¹ and R² may have a straight chain structure or may have a branched structure. For example, a methyl group, an ethyl group, a propyl group, an isopropyl group, an n-butyl group, an isobutyl group, a sec-butyl group, a t-butyl group, an n-pentyl group, an n-hexyl group, an n-heptyl group, an n-octyl group, an n-nonyl group, an n-decyl group, an n-undecyl group, and an n-dodecyl group are included.

The aryl group having 6 to 20 carbon atoms of the aryl group having 6 to 20 carbon atoms that may have a substituent of R¹ and R² includes, for example, a phenyl group, a 1-naphthyl group, and a 2-naphthyl group.

The substituent of the alkyl group having 1 to 20 carbon atoms that may have a substituent of R¹ and R² includes, for example: a halogen atom, such as a fluorine atom, a chlorine atom, and a bromine atom; an alkoxy group having 1 to 6 carbon atoms, such as an methoxy group, an ethoxy group, and a propoxy group; an aryloxy group having 6 to 20 carbon atoms, such as a phenoxy group, a 1-naphthyloxy group, and a 2-naphthyloxy group; an aryl group having 6 to 20 carbon atoms that may have a substituent, such as a phenyl group, a 4-fluoro-phenyl group, a 3-fluoro-phenyl group, a 2-fluoro-phenyl group, a 4-chloro-phenyl group, a 4-methyl-phenyl group, a 4-trifluoromethyl phenyl group, a 3-trifluoromethyl phenyl group, a 2-trifluoromethyl phenyl group, a 4-methoxyphenyl group, a 3-methoxyphenyl group, a 2-methoxyphenyl group, a 1-naphthyl group, and a 2-naphthyl group; and a cycloalkyl group having 3 to 20 carbon atoms, such as a cyclopropyl group, a cyclopentyl group, and a cyclohexyl group.

The substituent of the aryl group having 6 to 20 carbon atoms that may have a substituent of R¹ and R² includes, for example: a halogen atom, such as a fluorine atom, a chlorine atom, and a bromine atom; an alkyl group having 1 to 6 carbon atoms, such as a methyl group, an ethyl group, and a propyl group; an alkoxy group having 1 to 6 carbon atoms, such as a methoxy group, an ethoxy group, and a propoxy group; an aryloxy group having 6 to 20 carbon atoms, such as a phenoxy group, a 1-naphthyloxy group, and a 2-naphthyloxy group; an aryl group having 6 to 20 carbon atoms that may have a substituent, such as a phenyl group, a 4-chloro-phenyl group, a 2-chloro-phenyl group, a 2, 4-dichlorophenyl group, a 4-methyl-phenyl group, a 2-methyl-phenyl group, a 4-trifluoromethyl phenyl group, a 4-methoxyphenyl group, a 4-trifluoromethoxyphenyl group, a 2-methoxyphenyl group, a 2, 4-dimethoxyphenyl group, a 4-cyanophenyl group, a 4-nitrophenyl group, a 4-methylthiophenyl group, a 1-naphthyl group, and a 2-naphthyl group; and a cycloalkyl group having 3 to 20 carbon atoms, such as a cyclopropyl group, a cyclopentyl group, and a cyclohexyl group.

In the present invention, preferably, at least one of R¹ and R² is a hydrogen atom, and more preferably, both R¹ and R² are hydrogen atoms.

Each of R³ and R⁴ independently represents a hydrogen atom, an alkyl group having 1 to 20 carbon atoms that may have a substituent, a cycloalkyl group having 3 to 20 carbon atoms that may have a substituent, or an aryl group having 6 to 20 carbon atoms that may have a substituent.

Where a case where both R³ and R⁴ are hydrogen atoms is excluded.

The alkyl group having 1 to 20 carbon atoms that may have a substituent of R³ and R⁴ includes similar ones that have been exemplified as the alkyl group having 1 to 20 carbon atoms of the alkyl group having 1 to 20 carbon atoms that may have a substituent of R¹ and R² described above.

The cycloalkyl group having 3 to 20 carbon atoms of the cycloalkyl group having 3 to 20 carbon atoms that may have a substituent of R³ and R⁴ includes, for example, a cyclopropyl group, a cyclopentyl group, a cyclohexyl group, and a cyclooctyl group.

The aryl group having 6 to 20 carbon atoms that may have a substituent of R³ and R⁴ includes similar ones that have been exemplified as the aryl group having 6 to 20 carbon atoms that may have a substituent of R¹ and R² described above.

The substituent of the alkyl group having 1 to 20 carbon atoms that may have a substituent of R³ and R⁴ includes similar ones that have been exemplified as the substituent of the alkyl group having 1 to 20 carbon atoms that may have a substituent of R¹ and R² described above.

The substituent of the cycloalkyl group having 3 to 20 carbon atoms that may have a substituent of R³ and R⁴ includes, for example: a halogen atom, such as a fluorine atom, a chlorine atom, and a bromine atom; an alkyl group having 1 to 6 carbon atoms, such as a methyl group, an ethyl group, and a propyl group; a haloalkyl group having 1 to 6 carbon atoms, such as a trifluoromethyl group; an alkoxy group having 1 to 6 carbon atoms, such as a methoxy group, an ethoxy group, and a propoxy group; an aryloxy group having 6 to 20 carbon atoms, such as a phenoxy group, a 1-naphthyloxy group, and a 2-naphthyloxy group; and an aryl group having 6 to 20 carbon atoms that may have a substituent, such as a phenyl group, a 4-chloro-phenyl group, a 4-methyl-phenyl group, a 2-methoxyphenyl group, a 1-naphthyl group, and a 2-naphthyl group.

The substituent of the aryl group having 6 to 20 carbon atoms that may have a substituent of R³ and R⁴ include similar ones that have been exemplified as the substituent of the aryl group having 6 to 20 carbon atoms that may have a substituent of R¹ and R².

R³ and R⁴ may form a ring [nitrogen-containing heterocycle containing nitrogen atoms of (a group represented by formula: -N(R³)(R⁴))] together.

In the present invention, a triazole urea compound represented by the formula (I) preferably has the group represented by the formula: -N(R³)(R⁴) in the formula (I) that is any one of the (B-1) to (B-7).

In the above-described formula (B-1), each of R^{a} and R^{b} independently represents an alkyl group having 1 to 20 carbon atoms that may have a substituent or an aryl group having 6 to 20 carbon atoms that may have a substituent.

The alkyl group having 1 to 20 carbon atoms that may have a substituent of R^{a} and R^{b} includes ones similar to the alkyl group having 1 to 20 carbon atoms that may have a substituent of R¹ and R² described above. The aryl group having 6 to 20 carbon atoms that may have a substituent of R^{a} and R^{b} includes ones similar to the aryl group having 6 to 20 carbon atoms that may have a substituent of R¹ and R² described above.

In the formulae (B-2), (B-3), (B-4), (B-5), (B-6) and (B-7), each of R^{c}, R^{d}, R^{e}, R^{g}, and R^{h} independently represents: a nitro group; a cyano group; a halogen atom, such as a fluorine atom, a chlorine atom, and a bromine atom; an alkyl group having 1 to 6 carbon atoms, such as a methyl group, an ethyl group, a propyl group, and an isopropyl group; a haloalkyl group having 1 to 6 carbon atoms, such as a trifluoromethyl group; a cycloalkyl group having 3 to 20 carbon atoms, such as a cyclopropyl group, a cyclopentyl group, and a cyclohexyl group; an alkoxy group having 1 to 6 carbon atoms, such as a methoxy group, an ethoxy group, and a propoxy group; an amino group; a mono-substituted amino group, such as a methylamino group, a phenylamino group, and an acetylamino group; a disubstituted amino group, such as a dimethylamino group, a diethylamino group, a phenylmethylamino group, and an acetylmethylamino group; an alkylthio group having 1 to 6 carbon atoms, such as a methylthio group, an ethylthio group, and a propylthio group; an alkylsulfenyl group having 1 to 6 carbon atoms, such as a methylsulfenyl group, an ethylsulfenyl group, and a propylsulfenyl group; an alkylsulfonyl group having 1 to 6 carbon atoms, such as a methylsulfonyl group, an ethylsulfonyl group, and a propylsulfonyl group; an alkoxycarbonyl group having 2 to 10 carbon atoms, such as a methoxy carbonyl group and an ethoxy carbonyl group; an alkylcarbonyl group having 2 to 10 carbon atoms, such as an acetyl group and a propionyl group; or an aryl group having 6 to 20 carbon atoms that may have a substituent, such as a phenyl group, a 4-chloro-phenyl group, a 4-methyl-phenyl group, a 2-methoxyphenyl group, a 4-trifluoromethyl phenyl group, a 1-naphthyl group, and a 2-naphthyl group.

Among these, R^{c}, R^{d}, R^{e}, R^{g}, and R^{h} are preferably a nitro group, a cyano group, a halogen atom, an alkyl group having 1 to 6 carbon atoms, a haloalkyl group having 1 to 6 carbon atoms, an alkoxy group having 1 to 6 carbon atoms and an aryl group having 6 to 20 carbon atoms that may have a substituent.

R^{f} represents a hydrogen atom, an alkyl group having 1 to 20 carbon atoms that may have a substituent, a cycloalkyl group having 3 to 20 carbon atoms that may have a substituent, an aryl group having 6 to 20 carbon atoms that may have a substituent, a heterocyclic group that may have a substituent, or an acyl group having 2 to 20 carbon atoms.

Specific examples as the alkyl group having 1 to 20 carbon atoms of the alkyl group having 1 to 20 carbon atoms that may have a substituent and the aryl group having 6 to 20 carbon atoms of the aryl group having 6 to 20 carbon atoms that may have a substituent of R^{f} include ones similar to the ones listed as the specific examples of the alkyl group having 1 to 20 carbon atoms of the alkyl group having 1 to 20 carbon atoms that may have a substituent and the aryl group having 6 to 20 carbon atoms of the aryl group having 6 to 20 carbon atoms that may have a substituent of R¹ and R² described above.

Specific examples of the cycloalkyl group having 3 to 20 carbon atoms of the cycloalkyl group having 3 to 20 carbon atoms that may have a substituent of R^{f} includes ones similar to the ones listed as the specific examples of the cycloalkyl group having 3 to 20 carbon atoms of the cycloalkyl group having 3 to 20 carbon atoms that may have a substituent of R³ and R⁴ described above.

The heterocyclic group of the heterocyclic group that may have the substituent of R^{f} is not specifically limited, and includes, for example: a five-membered heterocyclic group, such as a 2-furyl, a 3-furyl, an oxazol-2-yl, an oxazol-4-yl, an oxazol-5-yl, an isoxazol-3-yl, an isoxazol-4-yl, an isoxazol-5-yl, a 1, 2, 4-oxadiazol-3-yl, a 1, 2, 4-oxadiazol-5-yl, a 1, 3, 4-oxadiazol-2-yl, a thiazol-2-yl, a thiazol-4-yl, a thiazol-5-yl, an isothiazol-3-yl, an isothiazol-4-yl, an isothiazol-5-yl, a 1, 2, 4-thiadiazol-3-yl, a 1, 2, 4-thiadiazol-5-yl, a 1, 3, 4-thiadiazol-2-yl, an imidazol-2-yl, an imidazol-4-yl, a 2-thienyl, a 3-thienyl, a pyrazol-3-yl, a pyrazol-4-yl, a 1, 2, 4-triazol-3-yl, a tetrazol-5-yl, a 4, 5-dihydroisoxazol-3-yl, a 4, 5-dihydroisoxazol-4-yl, and a 4, 5-dihydroisoxazol-5-yl; a six-membered heterocyclic group, such as a 2-pyridyl, a 3-pyridyl, a 4-pyridyl, a pyridazin-3-yl, a pyrazin-2-yl, and a triazin-2-yl; and a fused ring heterocyclic group, such as a benzothiazol-2-yl, a benzoxazol-2-yl, a benzothiazol-7-yl, a benzisothiazol-3-yl, a benzofurazan-4-yl, and a benzoxadiazol-4-yl.

The acyl group having 2 to 20 carbon atoms of R^{f} includes, for example: an alkylcarbonyl group having 2 to 20 carbon atoms, such as an acetyl group and a propionyl group; and an arylcarbonyl group having 6 to 20 carbon atoms, such as a benzoyl group.

The substituent of the alkyl group having 1 to 20 carbon atoms that may have a substituent of R^{f} described above includes: a halogen atom, such as a fluorine atom, a chlorine atom, and a bromine atom; an alkoxy group having 1 to 6 carbon atoms, such as a methoxy group, an ethoxy group, and a propoxy group; an aryloxy group having 6 to 20 carbon atoms, such as a phenoxy group, a 1-naphthyloxy group, and a 2-naphthyloxy group; an aryl group having 6 to 20 carbon atoms that may have a substituent, such as a phenyl group, a 4-fluoromethylphenyl group, a 3-fluoro-phenyl group, a 2-fluoro-phenyl group, a 4-chloro-phenyl group, a 4-methyl-phenyl group, a 4-trifluoromethyl phenyl group, a 3-trifluoromethyl phenyl group, a 2-trifluoromethyl phenyl group, a 4-methoxyphenyl group, a 3-methoxyphenyl group, a 2-methoxyphenyl group, a 1-naphthyl group, and a 2-naphthyl group; and a cycloalkyl group having 3 to 20 carbon atoms, such as a cyclopropyl group, a cyclopentyl group, and a cyclohexyl group.

The substituents of the cycloalkyl group having 3 to 20 carbon atoms that may have a substituent, the aryl group having 6 to 20 carbon atoms that may have a substituent, and the heterocyclic group that may have a substituent of R^{f} described above includes, for example: a halogen atom, such as a fluorine atom, a chlorine atom, and a bromine atom; an alkyl group having 1 to 6 carbon atoms, such as a methyl group, an ethyl group, and an isopropyl group; an alkoxy group having 1 to 6 carbon atoms, such as a methoxy group, an ethoxy group, and a propoxy group; an aryloxy group having 6 to 20 carbon atoms, such as a phenoxy group, a 1-naphthyloxy group, and a 2-naphthyloxy group; an aryl group having 6 to 20 carbon atoms that may have a substituent, such as a phenyl group, a 4-chloro-phenyl group, a 4-methyl-phenyl group, a 2-methoxyphenyl group, a 1-naphthyl group, and a 2-naphthyl group; and a cycloalkyl group having 3 to 20 carbon atoms, such as a cyclopropyl group, a cyclopentyl group, and a cyclohexyl group.

Among these, R^{f} is preferably an alkyl group having 1 to 20 carbon atoms that may have a substituent, an aryl group having 6 to 20 carbon atoms that may have a substituent, or a heterocyclic group that may have a substituent.

Each of *p, q, r, s, t* independently represents an integer of 0 to 4, each of them is preferred to be 0 or 1 independently.

Specific examples of the triazole urea compound represented by the formula (I) include the following, but not limited to these.

A compound in which the group represented by -N(R³)(R⁴) in the formula (I) is the group represented by the formula (B-1), such as 1-(dimethylaminocarbonyl)-1,2,3-triazole, 1-(diethylaminocarbonyl)-1,2,3-triazole, 1-(dipropylaminocarbonyl)-1,2,3-triazole, 1-(diisopropylaminocarbonyl)-1,2,3-triazole, 1-(dibutylaminocarbonyl)-1,2,3-triazole, 1-(dipentylaminocarbonyl)-1,2,3-triazole, 1-(benzylmethylaminocarbonyl)-1,2,3-triazole, 1-(methylphenylaminocarbonyl)-1,2,3-triazole, 2-(dimethylaminocarbonyl)-1,2,3-triazole, 2-(diethylaminocarbonyl)-1,2,3-triazole, 2-(dipropylaminocarbonyl)-1,2,3-triazole, 2-(diisopropylaminocarbonyl)-1,2,3-triazole, 2-(dibutylaminocarbonyl)-1,2,3-triazole, 2-(dipentylaminocarbonyl)-1,2,3-triazole, 2-(benzylmethylaminocarbonyl)-1,2,3-triazole, and 2-(methylphenylaminocarbonyl)-1,2,3-triazole;
a compound in which the group represented by -N(R³)(R⁴) in the formula (I) is the group represented by the formula (B-2), such as 1-(pyrrolidine-1'-yl)carbonyl-1,2,3-triazole, 1-(2-methyl pyrrolidine-1'-yl)carbonyl-1,2,3-triazole, 1-(3-methylpyrrolidine-1'-yl)carbonyl-1,2,3-triazole, 1-(2,5-dimethylpyrrolidine-1'-yl)carbonyl-1,2,3-triazole, 1-(3,4-dimethylpyrrolidine-1'-yl)carbonyl-1,2,3-triazole, 1-(3-phenylpyrrolidine-1'-yl)carbonyl-1,2,3-triazole, 2-(pyrrolidine-1'-yl)carbonyl-1,2,3-triazole, 2-(2-methylpyrrolidine-1'-yl)carbonyl-1,2,3-triazole, 2-(3-methylpyrrolidine-1'-yl)carbonyl-1,2,3-triazole, 2-(2,5-dimethylpyrrolidine-1'-yl)carbonyl-1,2,3-triazole, 2-(3,4-dimethylpyrrolidine-1'-yl)carbonyl-1,2,3-triazole, and 2-(3-phenylpyrrolidine-1'-yl)carbonyl-1,2,3-triazole;
a compound in which the group represented by -N(R³)(R⁴) in the formula (I) is the group represented by the formula (B-3), such as 1-(piperidine-1'-yl)carbonyl-1,2,3-triazole, 1-(2-methylpiperidine-1'-yl)carbonyl-1,2,3-triazole, 1-(3-methylpiperidine-1'-yl)carbonyl-1,2,3-triazole, 1-(2,5-dimethyl piperidine-1'-yl)carbonyl-1,2,3-triazole, 1-(3,4-dimethyl piperidine-1'-yl)carbonyl-1,2,3-triazole, 1-(4-phenylpiperidine-1'-yl)carbonyl-1,2,3-triazole, 2-(piperidine-1'-yl)carbonyl-1,2,3-triazole, 2-(2-methylpiperidine-1'-yl)carbonyl-1,2,3-triazole, 2-(3-methylpiperidine-1'-yl)carbonyl-1,2,3-triazole, 2-(2,5-dimethyl piperidine-1'-yl)carbonyl-1,2,3-triazole, 2-(3,4-dimethyl piperidine-1'-yl)carbonyl-1,2,3-triazole, and 2-(4-phenylpiperidine-1'-yl)carbonyl-1,2,3-triazole;
a compound in which the group represented by -N(R³)(R⁴) in the formula (I) is the group represented by the formula (B-4), such as 1-(morpholine-1'-yl)carbonyl-1,2,3-triazole, 1-(2-methylmorpholine-1'-yl)carbonyl-1,2,3-triazole, 1-(3-methylmorpholine-1'-yl)carbonyl-1,2,3-triazole, 1-(2,5-dimethylmorpholine-1'-yl)carbonyl-1,2,3-triazole, 1-(3,4-dimethylmorpholine-1'-yl)carbonyl-1,2,3-triazole, 1-(3-phenylmorpholine-1'-yl)carbonyl-1,2,3-triazole, 2-(morpholine-1'-yl)carbonyl-1,2,3-triazole, 2-(2-methylmorpholine-1'-yl)carbonyl-1,2,3-triazole, 2-(3-methylmorpholine-1'-yl)carbonyl-1,2,3-triazole, 2-(2,5-dimethylmorpholine-1'-yl)carbonyl-1,2,3-triazole, 2-(3,4-dimethylmorpholine-1'-yl)carbonyl-1 ,2,3-triazole, 1-(3'-phenylmorpholine-1'-yl)carbonyl-1,2,3-triazole, and 2-(3'-phenylmorpholine-1'-yl)carbonyl-1,2,3-triazole;
a compound in which the group represented by -N(R³)(R⁴) in the formula (I) is the group represented by the formula (B-5), such as 1-(piperazine-1'-yl)carbonyl-1,2,3-triazole, 1-(4'-methyl piperazine-1'-yl)carbonyl-1,2,3-triazole, 1-(4'-benzyl piperazine-1'-yl)carbonyl-1,2,3-triazole, 1-(4'-t-butyl piperazine-1'-yl)carbonyl-1,2,3-triazole, 1-(4'-diphenylmethylpiperazine-1'-yl)carbonyl-1,2,3-triazole, 1-[4'-(4"-trifluoromethylbenzyl)piperazine-1'-yl]carbonyl-1,2,3-triazole, 1-[4'-(4"-fluorobenzyl)piperazine-1'-yl]carbonyl-1,2,3-triazole, 1-[4'-(3"-methoxybenzyl)piperazine-1'-yl]carbonyl-1,2,3-triazole, 1-[4'-(2"-chlorobenzyl)piperazine-1'-yl]carbonyl-1,2,3-triazole, 1-[4'-(oxazole-2"-yl)piperazine-1'-yl]carbonyl-1,2,3-triazole, 1-[4'-(thiazole-2"-yl)piperazine-1'-yl]carbonyl-1,2,3-triazole, 1-[4'-(benzoxazole-2"-yl)piperazine-1'-yl]carbonyl-1,2,3-triazole, 1-[4'-(benzothiazole-2"-yl)piperazine-1'-yl]carbonyl-1,2,3-triazole, 1-[4'-(5"-chlorobenzothiazole-2"-yl)piperazine-1'-yl]carbonyl-1,2,3-triazole, 1-[4'-(1",2"-benzisothiazole-3"-yl)piperazine-1'-yl]carbonyl-1,2,3-triazole, 1-(4'-phenylpiperazine-1'-yl)carbonyl-1,2,3-triazole, 1-[4'-(4"-chlorophenyl)piperazine-1'-yl]carbonyl-1,2,3-triazole, 1-[4'-(3"-chlorophenyl)piperazine-1'-yl]carbonyl-1,2,3-triazole, 1-[4'-(2"-chlorophenyl)piperazine-1'-yl]carbonyl-1,2,3-triazole, 1-[4'-(4"-trifluoromethoxyphenyl)piperazine-1'-yl]carbonyl-1,2,3-triazole, 1-[4'-(3"-trifluoromethoxyphenyl)piperazine-1'-yl]carbonyl-1,2,3-triazole, 1-[4'-(2"-trifluoromethoxyphenyl)piperazine-1'-yl]carbonyl-1,2,3-triazole, 1-[4'-(4"-nitrophenyl)piperazine-1'-yl]carbonyl-1,2,3-triazole, 1-[4'-(3"-nitrophenyl)piperazine-1'-yl]carbonyl-1,2,3-triazole, 1-[4'-(2"-nitrophenyl)piperazine-1'-yl]carbonyl-1,2,3-triazole, 1-[4'-(4"-cyanophenyl)piperazine-1'-yl]carbonyl-1,2,3-triazole, 1-[4'-(3"-cyanophenyl)piperazine-1'-yl]carbonyl-1,2,3-triazole, 1-[4'-(2"-cyanophenyl)piperazine-1'-yl]carbonyl-1,2,3-triazole, 1-[4'-(benzoylpiperazine)-1'-yl]carbonyl-1,2,3-triazole, 1-[4'-(acetylpiperazine)-1'-yl]carbonyl-1,2,3-triazole, 1-[4'-(2",4"-dichlorophenyl)piperazine-1'-yl]carbonyl-1,2,3-triazole, 1-[4'-(pyridine-2"-yl)piperazine-1'-yl]carbonyl-1,2,3-triazole, 1-[4'-(pyridine-3"-yl)piperazine-1'-yl]carbonyl-1,2,3-triazole, 1-[4'-(pyridine-4"-yl)piperazine-1'-yl]carbonyl-1,2,3-triazole, 1-[4'-(4"-trifluoromethylphenyl)piperazine-1'-yl]carbonyl-1,2,3-triazole, 1-[4'-(3"-trifluoromethylphenyl)piperazine-1'-yl]carbonyl-1,2,3-triazole, 1-[4'-(2"-trifluoromethylphenyl)piperazine-1'-yl]carbonyl-1,2,3-triazole, 1-[4'-(4"-methoxyphenyl)piperazine-1'-yl]carbonyl-1,2,3-triazole, 1-[4'-(3"-methoxyphenyl)piperazine-1'-yl]carbonyl-1,2,3-triazole, 1-[4'-(2"-methoxyphenyl)piperazine-1'-yl]carbonyl-1,2,3-triazole, 1-[4'-(2",4"-dimethoxyphenyl)piperazine-1'-yl]carbonyl-1,2,3-triazole, 1-[4'-(3",5"-dimethoxyphenyl)piperazine-1'-yl]carbonyl-1,2,3-triazole, 1-[4'-(2",6"-methoxyphenyl)piperazine-1'-yl]carbonyl-1,2,3-triazole, 1-(4'-cyclohexyl piperazine-1'-yl)carbonyl-1,2,3-triazole, 1-[(4'-cyclopentylpiperazine)-1'-yl]carbonyl-1,2,3-triazole,
2-(piperazine-1'-yl)carbonyl-1,2,3-triazole, 2-(4'-methyl piperazine-1 '-yl)carbonyl-1,2,3-triazole, 2-(4'-benzyl piperazine-1'-yl)carbonyl-1,2,3-triazole, 2-(4'-t-butyl piperazine-1'-yl)carbonyl-1,2,3-triazole, 2-(4'-diphenylmethylpiperazine-1'-yl)carbonyl-1,2,3-triazole, 2-[4'-(4"-trifluoromethylbenzyl)piperazine-1'-yl]carbonyl-1,2,3-triazole, 2-[4'-(4"-fluorobenzyl)piperazine-1'-yl]carbonyl-1,2,3-triazole, 2-[4'-(3"-methoxybenzyl)piperazine-1'-yl]carbonyl-1,2,3-triazole, 2-[4'-(4"-chlorobenzyl)piperazine-1'-yl]carbonyl-1,2,3-triazole, 2-[4'-(3"-chlorobenzyl)piperazine-1'-yl]carbonyl-1,2,3-triazole, 2-[4'-(2"-chlorobenzyl)piperazine-1'-yl]carbonyl-1,2,3-triazole, 1-[4'-(4"-trifluoromethoxyphenyl)piperazine-1'-yl]carbonyl-1,2,3-triazole, 1-[4'-(3"-trifluoromethoxyphenyl)piperazine-1'-yl]carbonyl-1,2,3-triazole, 1-[4'-(2"-trifluoromethoxyphenyl)piperazine-1'-yl]carbonyl-1,2,3-triazole, 2-(4'-benzoylpiperazine-1'-yl)carbonyl-1,2,3-triazole, 2-(4'-acetylpiperazine-1'-yl)carbonyl-1,2,3-triazole, 2-[4'-(oxazole-2"-yl)piperazine-1'-yl]carbonyl-1,2,3-triazole, 2-[4'-(thiazole-2"-yl)piperazine-1'-yl]carbonyl-1,2,3-triazole, 2-[4'-(benzoxazole-2"-yl)piperazine-1'-yl]carbonyl-1,2,3-triazole, 2-[4'-(benzothiazole-2"-yl)piperazine-1'-yl]carbonyl-1,2,3-triazole, 2-[4'-(5"-chlorobenzothiazole-2"-yl)piperazine-1'-yl]carbonyl-1,2,3-triazole, 2-[4'-(1",2"-benzisothiazole-3"-yl)piperazine-1'-yl]carbonyl-1,2,3-triazole, 2-(4'-phenylpiperazine-1'-yl)carbonyl-1,2,3-triazole, 2-[4'-(4"-chlorophenyl)piperazine-1'-yl]carbonyl-1,2,3-triazole, 2-[4'-(3"-chlorophenyl)piperazine-1'-yl]carbonyl-1,2,3-triazole, 2-[4'-(2"-chlorophenyl)piperazine-1'-yl]carbonyl-1,2,3-triazole, 2-[4'-(4"-nitrophenyl)piperazine-1'-yl]carbonyl-1,2,3-triazole, 2-[4'-(3"-nitrophenyl)piperazine-1'-yl]carbonyl-1,2,3-triazole, 2-[4'-(2"-nitrophenyl)piperazine-1'-yl]carbonyl-1,2,3-triazole, 2-[4'-(4"-cyanophenyl)piperazine-1'-yl]carbonyl-1,2,3-triazole, 2-[4'-(3"-cyanophenyl)piperazine-1'-yl]carbonyl-1,2,3-triazole, 2-[4'-(2"-cyanophenyl)piperazine-1'-yl]carbonyl-1,2,3-triazole, 2-[4'-(2",4"-dichlorophenyl)piperazine-1'-yl]carbonyl-1,2,3-triazole, 2-[4'-(pyridine-2"-yl)piperazine-1'-yl]carbonyl-1,2,3-triazole, 2-[4'-(pyridine-3"-yl)piperazine-1'-yl]carbonyl-1,2,3-triazole, 2-[4'-(pyridine-4"-yl)piperazine-1'-yl]carbonyl-1,2,3-triazole, 2-[4'-(4"-trifluoromethylphenyl)piperazine-1'-yl]carbonyl-1,2,3-triazole, 2-[4'-(3"-trifluoromethylphenyl)piperazine-1'-yl]carbonyl-1,2,3-triazole, 2-[4'-(2"-trifluoromethylphenyl)piperazine-1'-yl]carbonyl-1,2,3-triazole, 2-[4'-(4"-methoxyphenyl)piperazine-1'-yl]carbonyl-1,2,3-triazole, 2-[4'-(3"-methoxyphenyl)piperazine-1'-yl]carbonyl-1,2,3-triazole, 2-[4'-(2"-methoxyphenyl)piperazine-1'-yl]carbonyl-1,2,3-triazole, 2-[4'-(2",4"-dimethoxyphenyl)piperazine-1'-yl]carbonyl-1,2,3-triazole, 2-[4'-(3",5"-dimethoxyphenyl)piperazine-1'-yl]carbonyl-1,2,3-triazole, 2-[4'-(2",6"-dimethoxyphenyl)piperazine-1'-yl]carbonyl-1,2,3-triazole, 2-[(4'-cyclohexyl piperazine)-1'-yl]carbonyl-1,2,3-triazole, and 2-[(4'-cyclopentylpiperazine)-1'-yl]carbonyl-1,2,3-triazole;
a compound in which the group represented by -N(R³)(R⁴) in the formula (I) is the group represented by the formula (B-6), such as 1-(indoline-1'-yl)carbonyl-1,2,3-triazole, 1-(4-methylindoline-1'-yl)carbonyl-1,2,3-triazole, 1-(5-methylindoline-1'-yl)carbonyl-1,2,3-triazole, 1-(5-t-butyl indoline-1'-yl)carbonyl-1,2,3-triazole, 1-(2,4-dimethylindoline-1'-yl)carbonyl-1,2,3-triazole, 1-(5-phenylindoline-1'-yl)carbonyl-1,2,3-triazole, 2-(indoline-1'-yl)carbonyl-1,2,3-triazole, 2-(4-methylindoline-1'-yl)carbonyl-1,2,3-triazole, 2-(5-methylindoline-1'-yl)carbonyl-1,2,3-triazole, 2-(5-t-butyl indoline-1'-yl)carbonyl-1,2,3-triazole, 2-(2,4-dimethylindoline-1'-yl)carbonyl-1,2,3-triazole, 2-(5-phenylindoline-1'-yl)carbonyl-1,2,3-triazole, 1-(4-fluoroindoline-1'-yl)carbonyl-1,2,3-triazole, 1-(5-fluoroindoline-1'-yl)carbonyl-1,2,3-triazole, 1-(6-fluoroindoline-1'-yl)carbonyl-1,2,3-triazole, 1-(7-fluoroindoline-1'-yl)carbonyl-1,2,3-triazole, 1-(4-chloroindoline-1'-yl)carbonyl-1,2,3-triazole, 1-(5-chloroindoline-1'-yl)carbonyl-1,2,3-triazole, 1-(6-chloroindoline-1'-yl)carbonyl-1,2,3-triazole, 1-(7-chloroindoline-1'-yl)carbonyl-1,2,3-triazole, 1-(4-bromoindoline-1'-yl)carbonyl-1,2,3-triazole, 1-(5-bromoindoline-1'-yl)carbonyl-1,2,3-triazole, 1-(6-bromoindoline-1'-yl)carbonyl-1,2,3-triazole, 1-(7-bromoindoline-1'-yl)carbonyl-1,2,3-triazole, 1-(4-trifluoromethylindoline-1'-yl)carbonyl-1,2,3-triazole, 1-(5-trifluoromethylindoline-1'-yl)carbonyl-1,2,3-triazole, 1-(6-trifluoromethylindoline-1'-yl)carbonyl-1,2,3-triazole, 1-(7-trifluoromethylindoline-1'-yl)carbonyl-1,2,3-triazole, 2-(4-fluoroindoline-1'-yl)carbonyl-1,2,3-triazole, 2-(5-fluoroindoline-1'-yl)carbonyl-1,2,3-triazole, 2-(6-fluoroindoline-1'-yl)carbonyl-1,2,3-triazole, 2-(7-fluoroindoline-1'-yl)carbonyl-1,2,3-triazole, 2-(4-chloroindoline-1'-yl)carbonyl-1,2,3-triazole, 2-(5-chloroindoline-1'-yl)carbonyl-1,2,3-triazole, 2-(6-chloroindoline-1'-yl)carbonyl-1,2,3-triazole, 2-(7-chloroindoline-1'-yl)carbonyl-1,2,3-triazole, 2-(4-bromoindoline-1'-yl)carbonyl-1,2,3-triazole, 2-(5-bromoindoline-1'-yl)carbonyl-1,2,3-triazole, 2-(6-bromoindoline-1'-yl)carbonyl-1,2,3-triazole, 2-(7-bromoindoline-1'-yl)carbonyl-1,2,3-triazole, 2-(4-trifluoromethylindoline-1'-yl)carbonyl-1,2,3-triazole, 2-(5-trifluoromethylindoline-1'-yl)carbonyl-1,2,3-triazole, 2-(6-trifluoromethylindoline-1'-yl)carbonyl-1,2,3-triazole, and 2-(7-trifluoromethylindoline-1'-yl)carbonyl-1,2,3-triazole; and
a compound in which the group represented by -N(R³)(R⁴) in the formula (I) is the group represented by the formula (B-7), such as 1-(1',2',3',4'-tetrahydroquinoline-1'-yl)carbonyl-1,2,3-triazole, 1-(4'-methyl-1',2',3',4'-tetrahydroquinoline-1'-yl)carbonyl-1,2,3-triazole, 2-(4'-benzyl-1',2',3',4'-tetrahydroquinoline-1'-yl)carbonyl-1,2,3-triazole, 1-(4'-t-butyl-1',2',3',4'-tetrahydroquinoline-1'-yl)carbonyl-1,2,3-triazole, 1-(7'-methyl-1',2',3',4'-tetrahydroquinoline-1'-yl)carbonyl-1,2,3-triazole, 1-(7'-benzyl-1',2',3',4'-tetrahydroquinoline-1'-yl)carbonyl-1,2,3-triazole, 1-(7'-t-butyl-1',2',3',4'-tetrahydroquinoline-1'-yl)carbonyl-1,2,3-triazole, 1-(7'-phenyl-1',2',3',4'-tetrahydroquinoline-1'-yl)carbonyl-1,2,3-triazole, 2-(5'-methyl-1',2',3',4'-tetrahydroquinoline-1'-yl)carbonyl-1,2,3-triazole, 1-(6'-t-butyl-1',2',3',4'-tetrahydroquinoline-1'-yl)carbonyl-1,2,3-triazole, 2-(1',2',3',4'-tetrahydroquinoline-1'-yl)carbonyl-1,2,3-triazole, 2-(4'-methyl-1',2',3',4'-tetrahydroquinoline-1'-yl)carbonyl-1,2,3-triazole, 2-(4'-benzyl-1',2',3',4'-tetrahydroquinoline-1'-yl)carbonyl-1,2,3-triazole, 2-(4'-t-butyl-1',2',3',4'-tetrahydroquinoline-1'-yl)carbonyl-1,2,3-triazole, 2-(7'-methyl-1',2',3',4'-tetrahydroquinoline-1'-yl)carbonyl-1,2,3-triazole, 2-(7'-benzyl-1',2',3',4'-tetrahydroquinoline-1'-yl)carbonyl-1,2,3-triazole, 2-(7'-t-butyl-1',2',3',4'-tetrahydroquinoline-1'-yl)carbonyl-1,2,3-triazole, 2-(7'-phenyl-1',2',3',4'-tetrahydroquinoline-1'-yl)carbonyl-1,2,3-triazole, 2-(5'-methyl-1',2',3',4'-tetrahydroquinoline-1'-yl)carbonyl-1,2,3-triazole, and 2-(6'-t-butyl-1',2',3',4'-tetrahydroquinoline-1'-yl)carbonyl-1,2,3-triazole.

Among these, from an aspect of having an excellent strigolactone receptor inhibition activity, the group represented by -N(R³)(R⁴) in the formula (I) is preferably, a compound that is the group represented by the formulae (B-1), (B-2), (B-3), (B-4), (B-5) and (B-6), and more preferably, (i) a compound in which the group represented by -N(R³)(R⁴) in the formula (I) is the group represented by the formula (B-1), and R³ and R⁴ are an alkyl group having 1 to 6 carbon atoms that may have a substituent; (ii) a compound in which the group represented by -N (R³)(R⁴) in the formula (I) is the group represented by the formula (B-2); (iii) a compound in which the group represented by -N (R³)(R⁴) in the formula (I) is the group represented by the formula (B-4); (iv) a compound in which the group represented by -N (R³)(R⁴) in the formula (I) is the group represented by the formula (B-5); and (v) a compound in which the group represented by -N (R³)(R⁴) in the formula (I) is the group represented by the formula (B-6), and yet more preferably, the group represented by -N(R³)(R⁴) in the formula (I) is the group represented by the (B-6).

Among these, the following compounds are especially preferred. 1-(diethylaminocarbonyl)-1,2,3-triazole, 2-(diethylaminocarbonyl)-1,2,3-triazole, 1-(morpholine-1'-yl)carbonyl-1,2,3-triazole, 2-(morpholine-1'-yl)carbonyl)-1,2,3-triazole, 1-(methylphenylaminocarbonyl)-1,2,3-triazole, 2-(methylphenylaminocarbonyl)-1,2,3-triazole, 1-(1',2',3',4'-tetrahydroquinoline-1'-yl)carbonyl-1,2,3-triazole, 2-(1',2',3',4'-tetrahydroquinoline-1'-yl)carbonyl-1,2,3-triazole, 1-(pyrrolidine-1'-yl)carbonyl-1,2,3-triazole, 2-(pyrrolidine-1'-yl)carbonyl-1,2,3-triazole, 1-(indoline-1'-yl)carbonyl-1,2,3-triazole, 2-(indoline-1'-yl)carbonyl-1,2,3-triazole, 1-(4-fluoroindoline-1'-yl)carbonyl-1,2,3-triazole, 2-(4-fluoroindoline-1'-yl)carbonyl-1,2,3-triazole, 1-(5-fluoroindoline-1'-yl)carbonyl-1,2,3-triazole, 2-(5-fluoroindoline-1'-yl)carbonyl-1,2,3-triazole, 1-(6-fluoroindoline-1'-yl)carbonyl-1,2,3-triazole, 2-(6-fluoroindoline-1'-yl)carbonyl-1,2,3-triazole, 1-(7-fluoroindoline-1'-yl)carbonyl-1,2,3-triazole, 2-(7-fluoroindoline-1'-yl)carbonyl-1,2,3-triazole, 2-(4'-phenylpiperazine-1'-yl)carbonyl-1,2,3-triazole, 1-[4'-(2"-chlorophenyl)piperazine-1'-yl]carbonyl-1,2,3-triazole, 2-[4'-(2"-phenyl)piperazine-1'-yl]carbonyl-1,2,3-triazole, 1-[4'-(2"-methoxyphenyl)piperazine-1'-yl]carbonyl-1,2,3-triazole, 2-[4'-(2"-methoxyphenyl)piperazine-1'-yl]carbonyl-1,2,3-triazole, 1-(4'-diphenylmethyl)piperazine-1'-yl]carbonyl-1,2,3-triazole, 2-(4'-diphenylmethyl)piperazine-1'-yl]carbonyl-1,2,3-triazole, 1-[4'-(2",4"-dimethoxyphenyl)piperazine-1'-yl]carbonyl-1,2,3-triazole, 2-[4'-(2",4"-dimethoxyphenyl)piperazine-1'-yl]carbonyl-1,2,3-triazole, 1-(4'-benzyl piperazine-1'-yl)carbonyl-1,2,3-triazole, 2-(4'-benzyl piperazine-1'-yl]carbonyl-1,2,3-triazole, 1-[4'-(4"-trifluoromethylbenzyl)piperazine-1'-yl]carbonyl-1,2,3-triazole, 2-[4'-(4"-trifluoromethylbenzyl)piperazine-1'-yl]carbonyl-1,2,3-triazole, 1-[4'-(4"-methylbenzyl)piperazine-1'-yl]carbonyl-1,2,3-triazole, 2-[4'-(4"-methylbenzyl)piperazine-1'-yl]carbonyl-1,2,3-triazole, 1-[4'-(4"-fluorobenzyl)piperazine-1'-yl]carbonyl-1,2,3-triazole, 2-[4'-(4"-fluorobenzyl)piperazine-1'-yl]carbonyl-1,2,3-triazole, 1-[(benzothiazole-2'-yl)piperazine-1'-yl]carbonyl-1,2,3-triazole, 2-[(benzothiazole-2'-yl)piperazine-1'-yl]carbonyl-1,2,3-triazole, 1-[4'-(2"-trifluoromethylphenyl)piperazine-1'-yl]carbonyl-1,2,3-triazole, and 2-[4'-(2"-trifluoromethylphenyl)piperazine-1'-yl]carbonyl-1,2,3-triazole.

The compound represented by the above-described formula (I) is possible to form an acid addition salt, and can form the acid addition salt corresponding to a kind of the substituent. The kind of salt is not specifically limited, and include, for example: a salt with mineral acids, such as hydrochloric acid and sulfuric acid; a salt with organic acids, such as p-toluenesulfonic acid, methanesulfonic acid, and tartaric acid; a metal salt, such as a sodium salt, a potassium salt, and a calcium salt; a salt with organic amine, such as ammonium salt and triethylamine; and a salt with amino acid, such as glycine. Furthermore, the compound represented by the above-described formula (I) or its salt exists as a hydrate or a solvate in some cases. These substances may be used as an active ingredient of the inhibitor of the present invention.

Among the triazole urea compounds represented by the formula (I), the triazole urea compound represented by the formula (I-1) is a novel compound.

In (B¹-5) in the formula (I-1), Rⁱ represents a hydrogen atom, an alkyl group having 1 to 20 carbon atoms that may have a substituent, an aryl group having 6 to 20 carbon atoms that may have a substituent, or a heterocyclic group that may have a substituent.

The specific examples of the alkyl group having 1 to 20 carbon atoms that may have a substituent, the aryl group having 6 to 20 carbon atoms that may have a substituent, or the heterocyclic group that may have a substituent of Rⁱ described above include: ones similar to the ones listed as the specific examples of the alkyl group having 1 to 20 carbon atoms that may have a substituent, the aryl group having 6 to 20 carbon atoms that may have a substituent, and the heterocyclic group that may have a substituent of R^{f} described above.

Each of R^{j} and R^{k} described above independently represents a nitro group, a cyano group, a halogen atom, an alkyl group having 1 to 6 carbon atoms, a cycloalkyl group having 3 to 20 carbon atoms, an alkoxy group having 1 to 6 carbon atoms, an amino group, a mono-substituted amino group, a disubstituted amino group, an alkylthio group having 1 to 6 carbon atoms, an alkylsulfenyl group having 1 to 6 carbon atoms, an alkylsulfonyl group having 1 to 6 carbon atoms, an alkoxycarbonyl group having 2 to 10 carbon atoms, an alkylcarbonyl group having 2 to 10 carbon atoms, or an aryl group having 6 to 20 carbon atoms that may have a substituent.

The specific examples of the alkyl group having 1 to 6 carbon atoms, the cycloalkyl group having 3 to 20 carbon atoms, the alkoxy group having 1 to 6 carbon atoms, the mono-substituted amino group, the disubstituted amino group, the alkylthio group having 1 to 6 carbon atoms, the alkylsulfenyl group having 1 to 6 carbon atoms, the alkylsulfonyl group having 1 to 6 carbon atoms, the alkoxycarbonyl group having 2 to 10 carbon atoms, the alkylcarbonyl group having 2 to 10 carbon atoms, or the aryl group having 6 to 20 carbon atoms that may have a substituent of R^{j} and R^{k} described above include ones similar to the ones listed as the specific examples of the alkyl group having 1 to 6 carbon atoms, the cycloalkyl group having 3 to 20 carbon atoms, the alkoxy group having 1 to 6 carbon atoms, the mono-substituted amino group, the disubstituted amino group, the alkylthio group having 1 to 6 carbon atoms, the alkylsulfenyl group having 1 to 6 carbon atoms, the alkylsulfonyl group having 1 to 6 carbon atoms, the alkoxycarbonyl group having 2 to 10 carbon atoms, the alkylcarbonyl group having 2 to 10 carbon atoms, or the aryl group having 6 to 20 carbon atoms that may have a substituent of R^{f} and R^{g} described above.

Each of *u* and *v* independently represents an integer of 0 to 4, each of them is preferred to be 0 or 1 independently.

The most of the compounds represented by the formula (I-1) has an excellent strigolactone receptor inhibition activity.

### [Method for Manufacturing Triazole Urea Compound Represented by Formula (I)]

The triazole compound represented by the formula (I) can be manufactured by a conventionally-known method. For example, as shown below, reacting a carbamoyl halide compound represented by a formula (II) with a triazole compound represented by a formula (III) ensures obtaining the triazole urea compound represented by the formula (I) as an object.

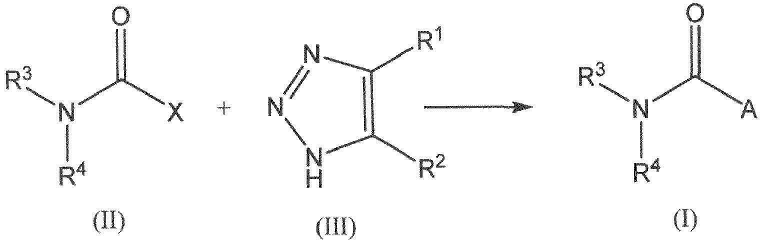

In the above-described formula, X represents a halogen atom, such as a chlorine atom and a bromine atom. R¹, R², R³, R⁴ and A represent meanings identical to the above.

The methods to execute the above-described reaction include, for example, (a) a method to add a predetermined amount of a base into an organic solvent solution of the carbamoyl halide compound represented by the formula (II) and the triazole compound represented by the formula (III), and stir the whole content at a predetermined temperature, and (b) a method to add a predetermined amount of the carbamoyl halide represented by the formula (II) or an organic solvent solution of the carbamoyl halide represented by the formula (II) into an organic solvent solution of the triazole compound represented by the formula (III) and a base, and stir the whole content at a predetermined temperature.

The used organic solvent is not specifically limited as long as it is an organic solvent that dissolves the carbamoyl halide represented by the formula (II) and the triazole compound represented by the formula (III) and is inactive to the reaction. For example, an ether solvent, such as diethyl ether, tetrahydrofuran, and 1, 3-dimethoxyethane; an amide solvent, such as N, N-dimethylformamide, N, N-dimethylacetamide, and N-methyl pyrrolidone; a halogenated hydrocarbon solvent, such as dichloromethane, chloroform, tetrachloromethane, and 1, 2-dichloroethane; nitrile solvent, such as acetonitrile and methacrylonitrile; an aromatic hydrocarbon solvent, such as benzene, toluene, and xylene; and a hydrocarbon solvent, such as pentane, hexane, and heptane are included. These can be used as one kind alone or as a combination of two kinds or more.

The used base includes, for example, a metallic hydroxide, such as sodium hydroxide and potassium hydroxide; a metal carbonate, such as sodium carbonate and potassium carbonate; a metal hydrogen carbonate, such as sodium hydrogen carbonate and potassium hydrogen carbonate; a metallic hydride, such as sodium hydride and potassium hydride; a metallic alkoxide, such as sodium methoxide, sodium ethoxide, potassium methoxide, potassium ethoxide, and magnesium ethoxide; and an organic base, such as triethylamine, N,N-diisopropylethylamine, pyridine, 4-(dimethylamino) pyridine, and a diazabicycloundecene. These can be used as one kind alone or as a combination of two kinds or more.

A usage of the carbamoyl halide compound represented by the formula (II) is usually 0.5 to 5 molar times, preferably 1.0 to 2 molar times with respect to one mol of the triazole compound represented by the formula (III).

A usage of the base is usually 0.5 to 3 molar times, preferably 1.0 to 2 molar times with respect to one mol of the carbamoyl halide compound represented by the formula (II).

The reaction is smoothly progressed within a temperature range from -10°C to a boiling point of the used solvent.

A reaction time is usually from a few minutes to a few hours.

After the reaction is finished, an object can be isolated by an ordinary post-processing operation in the organic chemistry.

With the above-described reaction, the triazole urea compound represented by the formula (I) as the object is usually obtained as a mixture of a compound in which A is the group represented by the (A-1) and a compound in which A is the group represented by the (A-2) in the formula (I).

The triazole compound represented by the formula (III) exists as a mixture of a tautomer of a compound represented by (III-1) and a compound represented by (III-2) below.

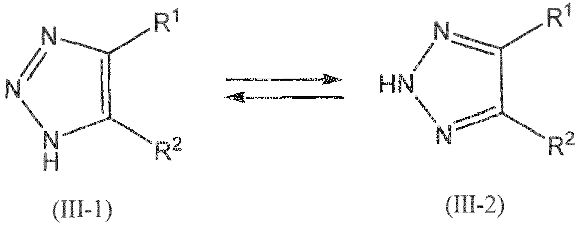

### (In the formula, R¹ and R² represent the meanings identical to the above.)

In view of this, when the reaction takes place in the state of the formula (III-1), the compound in which A is the group represented by the (A-1) in the formula (I) is obtained, and the reaction takes place in the state of the formula (III-2), the compound in which A is the group represented by the (A-2) in the formula (I) is obtained as the triazole urea compound represented by the formula (I) as the object. As a result, the mixture of the compound in which A is the group represented by the (A-1) and the compound in which A is the group represented by the (A-2) is obtained.

In the formula (I), the compound in which A is the group represented by the (A-1) and the compound in which A is the group represented by the (A-2) can be easily separated by known separation and purification means, such as column chromatography.

In the present invention, any one of the compound in which A is the group represented by the (A-1) in the formula (I), the compound in which A is the group represented by the (A-2) in the formula (I), and the mixture of the compound in which A is the group represented by the (A-1) and the compound in which A is the group represented by the (A-2) in the formula (I) can be used as the strigolactone receptor inhibitor of the present invention.

The strigolactone receptor inhibitor of the present invention irreversibly binds covalently to an amino acid residue in a hydrolysis activity center of a strigolactone of a receptor DWARF14 (D14). Therefore, it is considered that a strigolactone signal transmission is irreversibly inhibited when the strigolactone receptor inhibitor of the present invention is provided to a plant body.

### 2) Agricultural Composition

An agricultural composition of the present invention contains at least one kind of the triazole urea compounds represented by the formula (I) and an agriculturally acceptable formulation adjuvant.

The agriculturally acceptable formulation adjuvant used in the agricultural composition of the present invention is not specifically limited as long as it is agriculturally acceptable. For example, a surfactant, a binder, and a viscosity improver are included.

For example, a surfactant, such as an anionic surfactant of, for example, alkyl sulfate ester salt, alkyl aryl sulfonate, dialkyl sulfosuccinate, polyoxyethylene alkyl aryl ether phosphate ester salt, lignin sulfonate, and naphthalenesulfonate formaldehyde polycondensate, and a nonionic surfactant of, for example, polyoxyethylene alkyl ether, polyoxyethylene alkyl aryl ether, polyoxyethylene alkyl polyoxypropylene block copolymer, and sorbitan fatty acid ester;
and a binder or a viscosity improver of, for example, dextrin, sodium salt of carboxymethyl cellulose, polycarboxylic acid polymer compound, polyvinylpyrrolidone, polyvinyl alcohol, sodium lignin sulfonate, calcium lignin sulfonate, sodium polyacrylate, gum Arabic, sodium alginate, mannitol, sorbitol, bentonite mineral, polyacrylic acid and its derivative, sodium salt of carboxymethyl cellulose, white carbon, natural saccharide derivative (e.g., xanthan gum, guar gum) are included.

These surfactant, binder, and viscosity improver can be used as one kind alone or as a combination of two kinds or more.

The usage of the formulation adjuvant usually has a proportion of 0.1 to 50 weight%, preferably, approximately 0.1 to 25 weight% with respect to the whole composition.

The agricultural composition of the present invention is, for example, used by mixing at least one kind of the triazole urea compounds represented by the formula (I), the formulation adjuvant, and, as necessary, a liquid carrier, a solid carrier, a gaseous carrier, a surfactant, and the like to formulate into a wettable powder, a water dispersible granule, a flowable agent, granules, a dry flowable agent, an emulsifiable concentrate, an aqueous solution, an oil solution, a smoking agent, an aerosol agent, a micro capsule, or the like.

A content of the triazole urea compound represented by the formula (I) in these formulations is usually 0.1 to 99%, preferably, 0.2 to 90% in a weight ratio.

The used liquid carriers include, for example: water, alcohols (e.g., methanol, ethanol, 1-propanol, 2-propanol, and ethylene glycol), ketones (e.g., acetone and methyl ethyl ketone), ethers (e.g., dioxane, tetrahydrofuran, ethylene glycol monomethyl ether, diethylene glycol monomethyl ether, and propylene glycol monomethyl ether), aliphatic hydrocarbons (e.g., hexane, octane, cyclohexane, kerosene, fuel oil, and machine oil), aromatic hydrocarbons (e.g., benzene, toluene, xylene, solvent naphtha, and methylnaphthalene), halogenated hydrocarbons (e.g., dichloromethane, chloroform, and carbon tetrachloride), acid amides (e.g., dimethylformamide, dimethylacetamide, and N-methyl pyrrolidone), esters (e.g., ethyl acetate, butyl acetate, and fatty acid glycerin ester), nitriles (e.g., acetonitrile and propionitrile).

These liquid carriers can be used as one kind alone or as a combination of two kinds or more.

The used solid carriers include, for example: vegetable powder (e.g., soy flour, tobacco powder, flour, and wood flour), mineral powder (e.g., clays, such as kaolin, bentonite, acid clay, and clay, talcs, such as talcum powder and pyrophyllite powder, silicas, such as diatomaceous earth and mica powder), alumina, sulfur powder, activated charcoal, saccharide (e.g., lactose and glucose), inorganic salts (e.g., calcium carbonate and sodium bicarbonate), and a glass hollow body (a natural glass with air bubbles contained inside through a sintering process).

These solid carriers can be used as one kind alone or as a combination of two kinds or more.

A usage of the liquid carrier or the solid carrier has a proportion of, usually, 1 to 99 weight%, preferably, approximately 10 to 99 weight% with respect to the whole formulation.

The agricultural composition of the present invention can be simultaneously used by being mixed with or without being mixed with another fungicide, insecticide, acaricide, nematocide, herbicide, plant growth regulator, fertilizer, or soil conditioner.

The strigolactone receptor inhibitor or the agricultural composition of the present invention can be used as a branching enhancer for plants.

Conventionally, the strigolactone is known as a plant hormone that controls branching of plants and have a branching inhibition activity for plants. The strigolactone receptor inhibitor or the agricultural composition of the present invention has an effect of inhibiting an activity of the strigolactone. When the strigolactone receptor inhibitor or the agricultural composition of the present invention is provided to a plant body, a strigolactone signal transmission is irreversibly inhibited, and as a result, branching of the plant is increased. Therefore, the strigolactone receptor inhibitor or the agricultural composition of the present invention can be used as a branching enhancer for plants.

In particular, when the strigolactone receptor inhibitor or the agricultural composition of the present invention is provided to a gramineous plant, tillering of the gramineous plant is accelerated.

Therefore, the strigolactone receptor inhibitor or the agricultural composition of the present invention can be preferably used as a tillering accelerator for a gramineous plant.

With an acceleration of tillering, an increased rice yield is expected.

In this description, the term "tillering" means that a branch occurs and grows from a joint of a stem close to a root in a gramineous plant crop, however, this term must not be interpreted in a limited way in any meanings, and must be most broadly interpreted. Therefore, applying the inhibitor of the present invention to a rice plant, a sugarcane, and the like can also achieve an increased biomass due to the branching.

The strigolactone receptor inhibitor or the agricultural composition of the present invention can increase the number of flowers by increasing branching in a flowering plant, therefore, it is applicable as a flower number increasing agent for agriculture and horticulture. The flowering plant as the application target is not particularly limited, other than farm products, such as rice plants and tree-fruits, it can be applied to any flowering plants, such as horticultural plants including tulips, roses, and the like.

The plants to which the strigolactone receptor inhibitor or the agricultural composition of the present invention can be used as the branching enhancer for plants is not specifically limited.

For example, the plants include: vegetables, such as corn, rice plant, wheat, barley, rye, oat, sorghum, cotton, soybean, peanut, buckwheat, sugar beet, rapeseed, sunflower, sugarcane, tobacco; farm products, such as solanaceous vegetables (e.g., eggplant, tomato, green pepper, chili pepper, and potato), cucurbitaceous vegetables (e.g., cucumber, squash, zucchini, watermelon, and melon), cruciferous vegetables (e.g., daikon, turnip, horseradish, kohlrabi, napa cabbage, cabbage, leaf mustard, broccoli, and cauliflower), asteraceous vegetables (e.g., burdock, crown daisy, artichoke, and lettuce), liliaceous vegetables (green onion, onion, garlic, and asparagus), apiaceous vegetables (e.g., carrot, parsley, celery, and parsnip), chenopodiaceous vegetables (e.g., spinach and chard), lamiaceous vegetables (e.g., shiso, mint, and basil), strawberry, sweet potato, yamaimo, and taro; ornamental plants; foliage plants; tree-fruits, such as pome fruits (e.g., apple, pear, Japanese pear, Chinese quince, and quince), stone fruits (e.g., peach, plum, nectarine, Japanese apricot, yellow peach, apricot, and prune), citruses (e.g., citrus unshiu, orange, lemon, lime, and grapefruit), nuts (e.g., chestnut, walnut, hazel, almond, pistachio, cashew nut, and macadamia nut), berries (e.g., blueberry, cranberry, blackberry, and raspberry), grapes, persimmon, olive, loquat, banana, coffee, date palm, and coconut; trees other than the fruit trees, such as camellia sinensis, mulberry, flower and tree, street trees (ash, birch, dogwood, eucalyptus, ginkgo, lilac, maple, oak, poplar, Chinese redbud, Chinese sweet gum, sycamore, Japanese zelkova, Japanese thuja, Japanese fir, tsuga, juniper, pine, spruce, and Japanese yew).

A "crop" also includes: a crop to which a resistance against a mutant with a deleted biosynthesis gene of the strigolactone and a herbicide, such as a HPPD inhibitor, such as isoxaflutole, an ALS inhibitor, such as imazethapyr and thifensulfuron methyl, an EPSP synthase inhibitor, a glutamine synthetase inhibitor, and bromoxynil is given by a classical breeding method or a genetic modification technique; a crop that is made possible to synthesize, for example, a selective toxin and the like known as a bacillus using a genetic modification technique; and a crop to which a capability of producing an anti-pathogenic substance having a selective effect is given using a genetic modification technique.

### 3) Germination Inhibitor for Striga Seeds

The triazole urea compound represented by the formula (I) has a germination inhibitory effect for *Striga* seeds. Therefore, the triazole urea compound represented by the formula (I) is effective as an active ingredient of a germination inhibitor for *Striga* seeds.

*Striga* (English name: witchweed or witches weed) is an annual semi-parasitic plant that is distributed in tropical and subtropical areas in Africa, Asia, and Australia. *Striga* has many kinds, and the number of kinds is said to be 40 kinds or more. *Striga* is not specifically limited, and known *Strigas* are included. For example, *Striga hermonthica, Striga gesnerioides, Striga asiatica, Striga aequinoctialis, Striga angolensis, Striga angustifolia, Striga aspera, Striga bilabiata, Striga brachycalyx, Striga chrysantha, Striga dalzieli, Striga elegans, Striga forbesii, Striga gastonii, Striga gracillima, Striga hallaei, Striga hirsuta, Striga junodii, Striga klingii, Striga latericea, Striga lepidagathidis, Striga lutea, Striga macrantha, Striga passargei, Striga pinnatifida, Striga primuloides, Striga pubiflora,* and *Striga yemenica* are included.

*Striga* parastitizes corn, proso millet, sorghum, sugarcane, rice, beans, and the like. The host parasitized by *Striga* presents medical conditions like a growth failure, a wilt, a drought like a chlorosis, a nutrient deficiency, and a vascular failure. A substance secreted by a root of a host contains the strigolactone, and the substance is said to be a signal transmitting molecule that accelerates a germination of the seeds of *Striga.*

As described above, the triazole urea compound represented by the formula (I) has a strigolactone receptor inhibitory effect, and also has a germination inhibitory effect for *Striga* seeds.

A method for using the triazole urea compound represented by the formula (I) (or a composition containing the triazole urea compound represented by the formula (I) as an active ingredient) as a germination inhibitor for *Striga* seeds is not specifically limited as long as it has an aspect in which the above-described active ingredient and *Striga* seed can be in contact. For example, an aspect in which an agent of the present invention is, for example, sprayed, dropped, applied, or mixed in a soil including *Striga* seeds is included. More specifically, an aspect in which the triazole urea compound represented by the formula (I) or the composition is, for example, sprayed, dropped, applied, or mixed in a soil including *Striga* seeds is included.

An excellent germination inhibitory effect for *Striga* seeds can be obtained even in a case where a strigolactone analog (agonist of strigolactone) is coadministered with the triazole urea compound represented by the formula (I).

A dosage form of the agent of the present invention is not specifically limited as long as it has an agriculturally acceptable dosage form. For example, the dosage forms include a liquid agent, a solid agent, a powder agent, a granular agent, a granule, a wettable powder, a flowable agent, an emulsifiable concentrate, a paste agent, a dispersing agent, and the like are included.

The germination inhibitor of the present invention may be made only of an active ingredient (the compound represented by the formula (I) or its salt, hydrate, or solvate), or may include various kinds of additives corresponding to the dosage form, the application aspect, and the like in addition to these. A content ratio of the active ingredient in the agent of the present invention is not specifically limited. Specifically, 0.0001 to 100 weight%, preferably, approximately 0.01 to 50 weight% is exemplarily illustrated.

The additive is not specifically limited as long as it is agriculturally acceptable additive. For example, a carrier, a surfactant, a viscosity improver, an extending agent, a binder, vitamins, an antioxidant, a pH adjuster, a volatilization inhibitor, a dye, and the like are included.

The usage of the germination inhibitor of the present invention is not specifically limited as long as it is an amount that can inhibit the germination of *Striga* seed, and is usually approximately 5 kg to 20 kg per 10 are.

### EXAMPLE

Next, while the present invention will be further specifically described with working examples of a manufacturing example, a formulation example, a test example, and the like, the present invention is not limited only to these examples.

### (1) Synthesis of Triazole Urea Compound (I)

### Production of 1-(pyrrolidine-1'-yl)carbonyl-1,2,3-triazole (KOK1007-N1 isomer) and 2-(pyrrolidine-1'-yl)carbonyl-1,2,3-triazole (compound 5-N1, N2 isomer)

1H-1,2,3-triazole (62 mg), 1-pyrrolidine carbonyl chloride (100 mg), 4-(dimethylamino) pyridine (catalytic amount) were dissolved in a tetrahydrofuran/triethylamine mixed liquid (5:1, 1.5 mL), and were stirred at 40°C for 6 hours. After vacuuming and distilling the solvent, the concentrate was purified by a silica gel column chromatography (n-hexane: ethyl acetate (volume ratio) = 3:1 to 2:1), and N1 isomer (68 mg, white solid) and N2 isomer (36 mg, white solid) as title compounds were obtained.

Similarly, the following compounds were synthesized. The following shows physical values of the obtained triazole urea compounds. Note that the N1 isomer means the compound in which A is the group represented by (A-1) in the formula (I), and the N2 isomer means the compound in which A is the group represented by (A-2) in the formula (I).

**First table-1**

| | | |
|---|---|---|
| 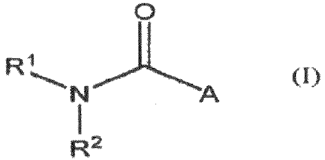 | | |
| ¹H NMR chemical shift values are listed in the following table. | | |
| Note that NMR was measured using deuterated chloroform unless otherwise specified (the same applies to the following) . | | |

| Compound No. | Structural formula | ¹H-NMR |
|---|---|---|
| Compound 1 N1 isomer | 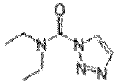 | *δ* = 7.80 (1H, s), 7.73 (1H, s), 3.59 (4H, s), 1.33 (6H, m). |
| Compound 1 N2 isomer | 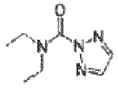 | *δ* = 7.80 (2H, s), 3.81-3.29 (4H, m), 1.29 (6H, t J = 7.1 Hz). |
| Compound 2 N1 isomer | 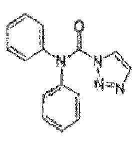 | *δ* = 7.60 (1H, d, *J* = 0.7 Hz), 7.57 (1H, d, *J* = 0.7 Hz), 7.38-7.16 (10H, m). |
| Compound 2 N2 isomer | 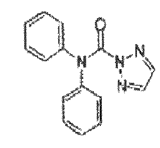 | *δ* = 7.52 (2H, s), 7.57 (1H, d, J = 0.7 Hz). 7.31-7.07 (10H, m). |
| Compound 3 N1 isomer | 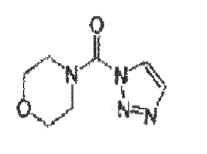 | *δ* = 7.84 (1H, s), 7.75 (1H, s), 4.02-3.80 (8H, m). |
| Compound 3 N2 isomer | 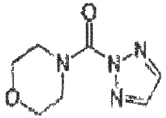 | *δ* = 7.83 (2H, s), 4.06-3.65 (8H, m). |

**First table-2**

| Compound No. | Structural formula | ¹H-NMR |
|---|---|---|
| Compound 4 N1 isomer | 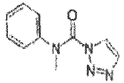 | *δ* = 7.53-7.47 (4H, m), 7.28-6.99 (3H, m), 3.50 (3H, s). |
| Compound 4 N2 isomer | 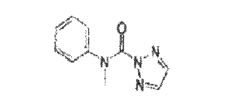 | *δ* = 7.58 (2H, s), 7.33-7.17 (3H, m), 7.10-7.04 (2H. m), 3.58 (3H, s). |
| Compound 5 N1 isomer | 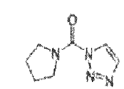 | *δ* = 7.82 (1H, d. *J* = C.9 Hz), 7.72 (1H, d *J* = 0.9 Hz), 3.76 (4H, m), 2.05-1.90 (4H, m). |
| Compound 5 N2 isomer | 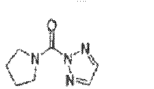 | *δ* = 7.74 (2H, s), 3.82 (2H, m), 3.67 (2H, m), 2.01-1.84 (4H, m). |
| Compound 6 N1 isomer | 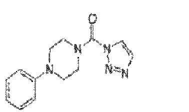 | *δ* = 8.21 (1H, d, J = 1.3 Hz), 7.75 (1H, d J = 1.3 Hz), 7.35-7.29 (2H, m), 6.99-6.90 (3H, m), 4.20-3.85 (4H, m). 3.40-3.27 (4H, m). |
| Compound 6 N2 isomer | 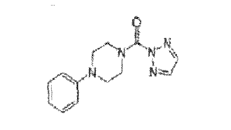 | *δ* = 7.84 (2H, s), 7.34-7.25 (3H, m), 6.98-6.89 (3H, m), 3.92 (4H, m), 3.31 (4H, m). |
| Compound 7 N1N2 isomer mixture | 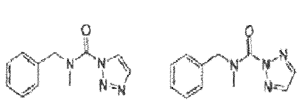 | N1 isomer |
| | | δ = 8.23 (1H, s), 7.74 (1H, d, J = 1.3 Hz), 7.45-7.25 (5H, m), 4.79 (2H, s), 3.26 (3H, s). N2 |
| | | isomer |
| | | 7.82 (2H, s), 7.45-7.25 (5H, m), 4.74 (2H, s), 3.07 (3H, s). |

**First table-3**

| | | |
|---|---|---|
| Compound 8 N1 isomer | 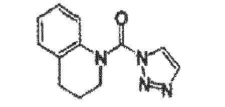 | *δ* = 8.12 (1H, d, J = 1.3 Hz), 7.72 (1H, d, *J* = 1.3 Hz), 7.24-6.92 (4H, m), 4.02 (2H, t, *J* = 6.3 Hz), 2.90 (2H, t, *J* = 6.8 Hz), 2.19-2.05 (2H, m). |
| Compound 8 N2 isomer | 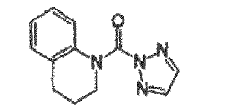 | *δ* = 7.73 (2H, s), 7.19 (1H, d, J = 7.7 Hz), 7.06 (1H, td, J = 7.7. 1.4 Hz), 6.96 (1H, td, J = 7.7, 1.4 Hz), 6.63 (1H, d, J = 7.7 Hz), 3.94 (2H, t, J = 6.6 Hz), 2.89 (2H, t, J = 6.6 Hz), 2.19-2.04 (2H, m). |
| Compound 9 N1, N2 isomer mixture | 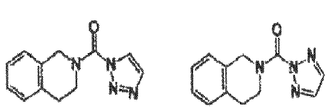 | N1 isomer |
| | | δ = 8.22 (1H, s), 7.75 (1H, d, J = 1.3 Hz), 7.30-7.14 (4H. m), 4.99-4.83 (2H, m), 4.19-3.70 (2H, m), 3.21-3.00 (2H, m), |
| | | N2 isomer |
| | | δ = 7.86 (2H, s), 7.30-7.13 (4H, m), 5.23-5.00 (2H, m), 4.19-3.70 (2H, m), 3.21-3.00 (2H, m). |
| Compound 10 N1 isomer | 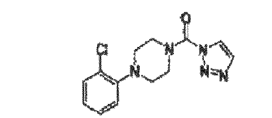 | *δ* = 8.21 (1H, d, J = 1.3 Hz), 7.75 (1H, d J = 1.3 Hz), 7.44-7.36 (1H, m), 7.30-7.21 (1H, m), 7.09-6.99 (2H, m), 4.23-3.88 (4H, m), 3.28-3.13 (4H, m). |
| Compound 10 N2 isomer | 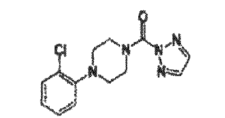 | *δ* = 7.84 (2H, s), 7.42-7.36 (1H, m), 7.28-7.21 (1H, m), 7.07-6.98 (2H, m), 4.06-3.81 (4H, m). 3.28-3.07 (4H, m). |
| Compound 11 N1 isomer | 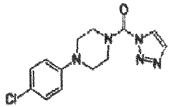 | *δ* = 8.21 (1H, d, J = 1.3 Hz), 7.75 (1H, d J = 1.3 Hz), 7.31-7.19 (2H, m), 6.92-6.82 (2H, m), 4.25-3.75 (4H, m), 3.37-3.21 (4H, m). |
| Compound 11 N2 isomer | 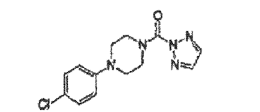 | *δ* = 7.85 (2H, s), 7.29-7.20 (2H, m). 6.91-6-82 (2H, m), 3.99-3.84 (4H, m), 3.35-3.19 (4H, m). |

**First table-4**

| | | |
|---|---|---|
| Compound 12 N1 isomer | 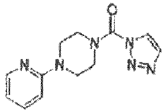 | *δ* = 8.25-8.19 (2H, m), 7.75 (1H, d, J = 1.3 Hz), 7.58-7.48 (1H, m), 6.74-6.64 (2H, m), 4.19-3.84 (4H. m), 3.82-3.64 (4H, m). |
| Compound 12 N2 isomer | 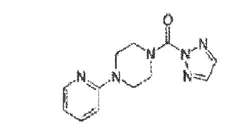 | *δ* = 8.24-8.19 (1H, m). 7.85 (2H, s), 7.58-7.48 (1H, m), 6.74-6.64 (2H. m), 3.95-3.82 (4H, m), 3.76-3.65 (4H, m). |
| Compound 13 N1 isomer | 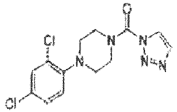 | *δ* = 8.21 (1H, d, J = 1.3 Hz), 7.75 (1H, d, J = 1.0 Hz), 7.41 (1H, d, J = 2.5 Hz), 7.23 (1H, dd, J = 8.6, 2.5 Hz), 6.98 (1H, d. J = 8.6 Hz), 4.22-3.88 (4H, m). 3.23-3.09 (4H, m). |
| Compound 13 N2 isomer | 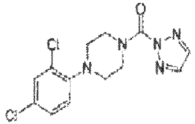 | *δ* = 7.84 (2H, s), 7.40 (1H, d, J = 2.3 Hz), 7.22 (1H, dd, J = 8.6, 2.3 Hz), 6.97 (1H, d, J = 8.6 Hz), 4.07-3.82 (4H, m), 3.26-3.05 (4H, m). |
| Compound 14 N1, N2 isomer mixture | 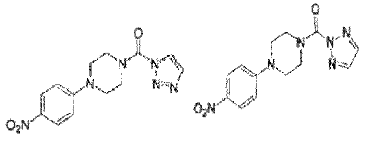 | N1 isomer |
| | | δ = 8.24 (1H, d. J = 1.3 Hz), 8.22-8.12 (2H, m), 7.77 (1H, d, J = 1.3 Hz), 6.92-6.82 (2H, m), 4.32-3.91 (4H, m), 3.69-3.53 (4H, m). |
| | | N2 isomer |
| | | δ = 8.22-8.12 (2H, m), 7.87 (2H, s), 6.92-6.82 (2H, m), 4.32-3.91 (4H, m), 3.69-3.53 (4H, m). |
| Compound 15 N1, N2 isomer mixture | 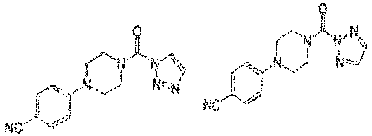 | N1 isomer |
| | | δ = 8.23 (1H, d, J = 1.3 Hz), 7.76 (1H, d, J = 1.3 Hz). 7.59-7.51 (2H, m), 6.94-6.87 (2H, m), 4.30-3.88 (4H, m), 3.59-3.43 (4H, m). |
| | | N2 isomer |
| | | δ = 7.86 (2H, s), 7.59-7.51 (2H, m), 6.94-6.87 (2H, m), 4.30-3.88 (4H, m), 3.59-3.43 (4H, m), |
| Compound 16 N1, N2 isomer mixture | 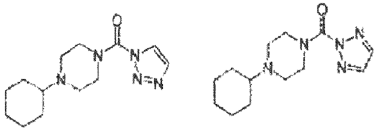 | N1 isomer |
| | | δ = 8.17 (1H, d. J = 1.3 Hz), 7.73 (1H, d, J = 1.3 Hz), 4.01-3.61 (4H, m). 2.81-2.59 (4H, m), 2.43-2.25 (2H. m), 1.93-1.75 (4H, m), 1.70-1.56 (1H, m), 1.36-0.99 (4H, m). |
| | | N2 isomer |
| | | δ = 7.82 (2H, s), 4.01-3.61 (4H. m), 2.81-2.59 (4H, m), 2.43-2.25 (2H, m), 1.93-1.75 (4H, m), 1.70-1.56 (1H, m), 1.36-0.99 (4H, m). |

**First table-5**

| | | |
|---|---|---|
| Compound 17 N1, N2 isomer mixture | 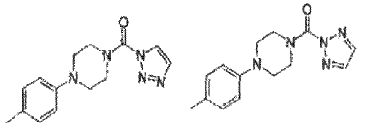 | N1 isomer |
| | | δ = 8.20 (1H, d. J = 1.0 Hz), 7.74 (1H, d, J = 1.0 Hz), 7.15-7.06 (2H, m). 6.90-6.82 (2H, m), 4.22-3.80 (4H, m), 3.35-3.16 (4H, m). 2.29 (3H, s). |
| | | N2 isomer |
| | | δ = 7.84 (1H, s), 7.15-7.06 (2H, m), 6.90-6.82 (2H, m), 4.22-3.80 (4H, m). 3.35-3.16 (4H, m), 2.29 (3H, s). |
| Compound 18 N1 isomer | 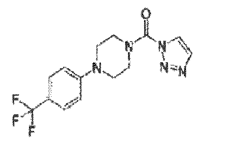 | *δ* = 8.22 (1H, d, J = 1.3 Hz), 7.76 (1H, d, J = 1.3 Hz), 7.53 (2H. d, J = 8.2 Hz), 6.96 (2H, d, J = 8.6 Hz), 4.28-3.85 (4H, m), 3.52-3.34 (4H, m). |
| Compound 18 N2 isomer | 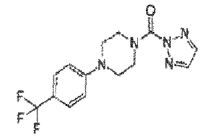 | *δ* = 7,86 (2H, s), 7.53 (2H, d, J = 8.2 Hz), 6.96 (2H, d, *J* = 8.6 Hz), 3.99-3.90 (4H, m), 3.49-3.36 (4H, m). |
| Compound 19 N1, N2 isomer mixture | 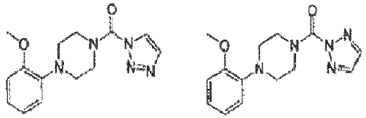 | N1 isomer |
| | | δ = 8.22 (1H, d, J = 1.3 Hz), 7.76 (1H, d, J = 1.3 Hz), 7.12-7.01 (1H, m), 6.99-6.87 (3H, m), 4.24-3.91 (4H, m), 3.89 (3H, s), 3.29-3.12 (4H, m). |
| | | N2 isomer |
| | | δ = 7.85 (2H. s), 7.12-7.01 (1H, m). 6.99-6.87 (3H, m), 4.24-3.91 (4H. m), 3.89 (3H, s), 3.29.3.12 (4H, m). |
| Compound 20 N1, N2 isomer mixture | 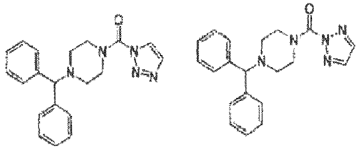 | N1 isomer |
| | | δ = 8.14 (1H, d. J = 1.3 Hz). 7.69 (1H, d, J = 1.3 Hz), 7.45-7.38 (4H, m). 7.33-7.15 (6H, m), 4.30 (1H, s), 4.07-3.60 (4H, m), 2.66-2.42 (4H, m). |
| | | N2 isomer |
| | | δ = 7.77 (2H, s), 7.45-7.38 (4H, m). 7.33-7.15 (6H, m), 4.29 (1H, s), 4.07-3.60 (4H, m). 2.66-2.42 (4H, m). |
| Compound 21 N1, N2 isomer mixture | 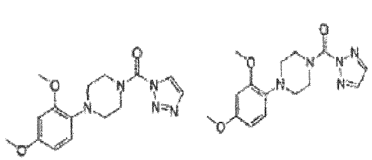 | N1 isomer |
| | | δ = 8.20 (1H, d, J = 1.3 Hz), 7.75 (1H, d, J = 1.3 Hz), 6.90-6.84 (1H, m). 6.53-6.49 (1H, m), 6.48-6.41 (1H, m), 4.22-3.88 (4H, m), 3.87 (3H, s). 3.79 (3H, s). 3.21-3.05 (4H, m). |
| | | N2 isomer |
| | | δ = 7.84 (2R, s), 6.90-6.84 (1H, m), 6.53-6.49 (1H, m), 6.48-6.41 (1H, m), 4.22-3.88 (4H, m), 3.86 (3H, s). 3.79 (3H, s). 3.21-3.05 (4H, m). |
| Compound 22 N1, N2 isomer mixture | 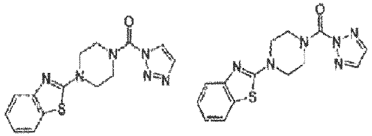 | N1 isomer |
| | | δ = 8.23 (1H, d. J = 1.3 Hz), 7.77 (3H, d. J = 1.3 Hz). 7.67-7.58 (2H, m), 7.38-7.29 (1H, m), 7.17-708 (1H, m), 4.24-3.75 (8H, m). |
| | | N2 isomer |
| | | δ = 7.87 (2H, s), 7.67-7.58 (2H, m), 7.38-7.29 (1 H, m), 7.17-7.08 (1H, m), 4.24-3.75 (8H, m). |

**First table-6**

| | | |
|---|---|---|
| Compound 23 N1, N2 isomer mixture | 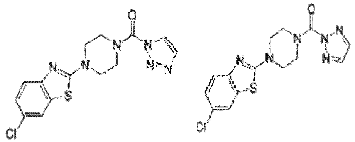 | N1 isomer |
| | | δ = 8.23 (1H, d, J = 1.3 Hz). 7.77 (1H, d, J = 1.3 Hz), 7.62-7.58 (1H, m), 7.49 (1H, dd, J = 8.4, 2.5 Hz), 7.32-7.25 (1H, m), 4.25-3.87 (4H, m), 3.87-373 (4H, m), |
| | | N2 isomer |
| | | δ = 7.87 (2H, s), 7.62-7.58 (1H, m), 7.49 (1H, dd, J = 8.4, 2.5 Hz), 7.32-7.25 (1H, m), 4.25-3.87 (4H, m), 3.87-3.73 (4H, m). |
| Compound 24 N1.N2 isomer mixture | 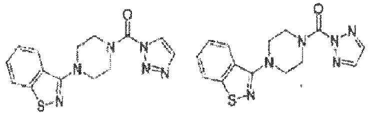 | N1 isomer |
| | | δ - 8.22 (1H, d, J - 1.3 Hz), 7.93-7.88 (1H, m), 7.87-7.82 (1H, m), 7.75 (1H, d, J = 1.3 Hz). 7.54-7.46 (1H, m), 7.43-7.35 (1H, m), 4.30-3.89 (4H, m), 3.79-3.58 (4H, m), |
| | | N2 isomer |
| | | δ = 7.93-7,88 (1H, m), 7.87-7.82 (3H, m), 7.54-7.46 (1H, m). 7.43-7.35 (1H, m), 4.30-3.89 (4H, m), 3.79-3.58 (4H, m), |
| Compound 25 N1 isomer | 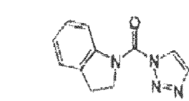 | *δ* = 8.36 (1H, d, J = 1.3 Hz). 8.11 (1H, br s), 7.78 (1H, d, J = 1.3 Hz), 7.35-7.27 (2H, m), 7.20-7.12 (1H, m), 4.67 (2H, t, J = 8.4 Hz), 3.27 (2H, t, J = 8.4 Hz). |
| Compound 25 N2 isomer | 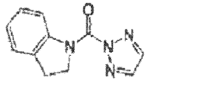 | *δ* = 8.05 (1H, br s), 7.87 (1H, s), 7.36-7.23 (2H, m), 7.16-7.08 (1H, m), 4.47 (2H, t, J = 8.4 Hz), 3.22 (2H, t, J = 8.4 Hz). |
| Compound 26 N1 isomer | 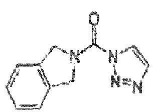 | *δ* = 8.40 (1H, d, J = 1.3 Hz), 7.78 (1H, d, J = 13 Hz). 7.39-7.28 (5H, m), 5.48 (2H. s), 5.12 (2H, s). |
| Compound 26 N2 isomer | 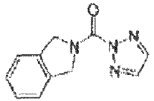 | *δ* = 7,89 (2H. s), 7.40-7.27 (4H, m), 5.32 (2H, s). 5.14 (2H, s). |

**First table-7**

| | | |
|---|---|---|
| Compound 27 N1, N2 isomer mixture | 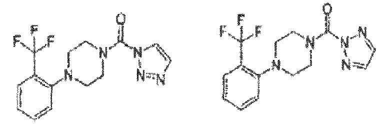 | N1 isomer |
| | | δ = 8.21 (1H, d, J = 1.3 Hz), 7.75 (1H, d, J = 1.3Hz), 7.67 (1H, d, J = 7.9 Hz), 7.56 (1H, t, J = 7.9 Hz), 7.37 (1H, d, J = 7.9 Hz). 7.30 (1H, t, J = 7.9 Hz), 4.21-3.80 (4H, m), 3.20-2.98 (4H, m), |
| | | N2 isomer |
| | | δ = 7.85 (2H, s), 7.67 (1H, d, J = 7.9 Hz), 7.56 (1H, t, J = 7.9 Hz), 7.37 (1H, d, J = 7.9 Hz). 7.30 (1H, t, J = 7.9 Hz). 4.21-3.80 (4H, m). 3.20-2.98 (4H, m). |
| Compound 28 N1, N2 isomer mixture | 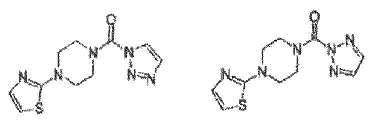 | N1 isomer |
| | | δ = 8.22 (1H, d, J = 1.3 Hz). 7.76 (1H, d, J = 1.3 Hz), 7.23 (1H, d, J = 3.6 Hz), 6.65 (1H, d. J = 3.6 Hz), 4.23-3.83 (4H, m), 3.76-3.60 (4H, m), |
| | | N2 isomer |
| | | δ = 7.86 (2H, s), 7.23 (1H, d. J = 3.5 Hz), 6.66 (1H, d, J = 3.5 Hz), 3.96-3.87 (4H, m), 3.71-3.62 (4H, m), |
| Compound 29 N1, N2 isomer mixture | 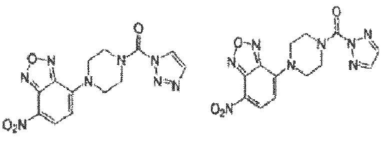 | DMSO-d6 |
| | | N1 isomer |
| | | δ = 8.61 (1H, d. J = 1.3 Hz), 8.56 (1H, d, J = 9.0 Hz). 7.96 (1H, d. J = 1.3 Hz), 6.61 (1H, d, J = 9.0 Hz). 4.46-4.20 (4H, m), 4.06-3.89 (4H, m). |
| | | N2 isomer |
| | | δ - 8.55 (1H, d, J - 8.9 Hz). 8.19 (2H, s), 6.61 (1H, d, J = 9.2 Hz), 4.46-4.20 (4H, m), 4.06-3.89 (4H, m), |
| Compound 30 N1 isomer | 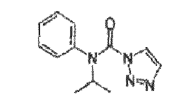 | *δ* = 7.95 (1H, d, J = 1.0 Hz), 7.49 (1H, d, J = 1.0 Hz), 7.37-7.28 (3H, m), 7.16-7.07 (2H, m), 4.96-4.79 (1H, m), 1.30 (6H, d, J = 6.9 Hz). |
| Compound 30 N2 isomer | 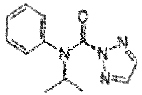 | *δ* = 7.49 (2H, s), 7.31-7.21 (3H, m), 7.13-7.04 (2H, m), 4.92-4.75 (1H, m), 1.31 (6H, d, J = 6.9 Hz), |
| Compound 31 N1 isomer | 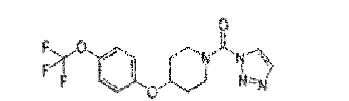 | *δ* = 8.17 (1H, d, J = 1.3 Hz), 7.74 (1H, d J = 1.3 Hz), 7.21-7.12 (2H, m). 6.97-6.89 (2H, m), 4.71-4.58 (1H, m), 4.13-3.77 (4H, m), 2.21-1.91 (4H, m), |
| Compound 31 N2 isomer | 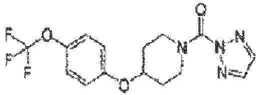 | *δ* = 7.82 (2H, s), 7.20-7.12 (2H, m), 6.97-6.89 (2H, m), 4.71-4.58 (1H, m), 4.13-3.59 (4H, m), 2,20-1.90 (4H, m). |
| Compound 32 N1, N2 isomer mixture | 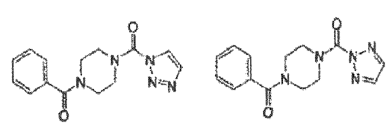 | N1 isomer |
| | | δ = 8.20 (1H, d. J = 1.3 Hz), 7.74 (1H, d, J = 1.3 Hz), 7.49-7.40 (5H, m), 4.12-3.49 (8H, m), |
| | | N2 isomer |
| | | δ = 7.84 (2H, s), 7.49-7.40 (5H, m), 4.12-3.49 (8H, m), |

**First table-8**

| | | |
|---|---|---|
| Compound 33 N1, N2 isomer | 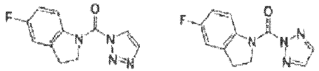 | N1 isomer |
| | | 8.37 (1H, d, J = 1.3 Hz), 814-8.02 (1H, m), 7.78 (1H, d. J = 1.3 Hz). 7.10-6.92 (2H, m), 4.71 (2H, t, J = 8.3 Hz). 3.26 (2H, t, J = 8.3 Hz). |
| | | N2 isomer |
| | | 8.17-7.96 (1H, m), 7.88 (2H, s), 7.03-6.91 (2H, m), 4.51 (2H, t, J = 8.4 Hz), 3.21 (2H, t, J = 8.4 Hz). |
| Compound 34 N1,N2 isomer | 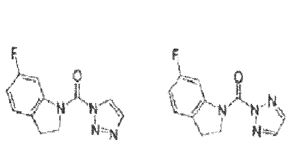 | N1 isomer |
| | | 8.37 (1H, d, J = 1.3 Hz). 7.98-7.81 (1H, m). 7.78 (1H, d, J = 1.3 Hz). 7.23-7.18 (1H, m), 6.86 (1H, td. J = 8.5, 2.5 Hz). 4.74 (2H, J = 8.4 Hz). 3.23 (2H, t. J = 8.4 Hz). |
| | | N2 isomer |
| | | 7.89 (2H, s), 7.87-7.68 (1H, m), 7.23-7.12 (1H, m), 6.83 (1H, td. J = 8.5, 2.4 Hz). 4.55 (2H. t, J = 8.4 Hz). 3.19 (2H, t, J = 8.4 Hz). |
| Compound 35 N1, N2 isomer | 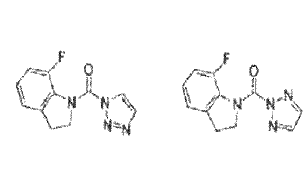 | N1 isomer |
| | | 8.31 (1H, d. J = 1.2 Hz). 7.76 (1H, d, J = 1.2 Hz). 7.19-6.91 (3H, m), 4.47 (2H, t, J = 7.8 Hz). 3.22 (2H, t, J = 7.8 Hz). |
| | | N2 isomer |
| | | 7.87 (2H, s), 7.17-7.02 (2H, m), 7.02-6.86 (1H, m). 4.34 (2H, t, J = 7.8 Hz). 3.21 (2H, t, J = 7.8 Hz). |
| Compound 36 N2 isomer | 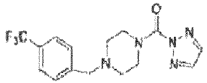 | N2 isomer |
| | | 7.82 (2H, s), 7.59 (2H, d, J = 8.0 Hz). 7.47 (2H, d, J = 8.0 Hz), 3.92-3.65 (4H, m), 3.61 (2H, s), 2.72-2.45 (4H, m). |

### (3) D14 Function Evaluation Test Using Yeast Two Hybrid System (Y2H)

Using the triazole urea compound synthesized above, a D14 function evaluation test using a yeast two hybrid system (Y2H) was performed. The Y2H is a system to detect an interaction between specific protein X: protein Y by using a transcription activator that has a separable DNA bonding and transcription activation domains, such as a yeast GAL4. In this system, the protein X is coupled to the DNA binding domain of the transcription activator, the protein Y is coupled to the transcription activation domain, and the DNA binding domain and the transcription activation domain align when the protein X and the protein Y interact, and thus, the transcription activator can operate.
In a yeast genome used in this system, a transcription activator response element and a reporter gene are coupled, and when the transcription activator binds to the response element, and the reporter gene is expressed, it is designed that the yeast can be grown in a certain kind of amino acid deficiency condition, or an expression of a specific enzyme is induced. Observing its growth and enzyme activity ensures detecting the interaction between the protein X: the protein Y.

In this test, D14 was coupled to the DNA binding domain of the transcription activator GAL4, and D53 (a protein that is an SL signal inhibitory factor) and SLR1 (a DELLA protein that is an inhibitory factor of gibberellin signal transmission) known to bind to D14 under an existence of the strigolactone was coupled to the transcription activation domain. Growing this yeast under the existence of the strigolactone activates a transcription of the reporter gene to ensure the growth even in a histidine, adenine deficient medium. By examining whether the yeast has grown or not in the histidine, adenine deficient medium when the strigolactone and the compound were simultaneously administered, an antagonist activity of the compound was evaluated.

As a result, a compound 1 (compound numbers correspond to the list in a first table. The same applies to the following), a compound 3, a compound 5, a compound 6, a compound 7, a compound 8, a compound 10, a compound 12, a compound 19, a compound 20, a compound 21, a compound 22, a compound 24, a compound 25, and a compound 27 indicated strong strigolactone antagonist activities.

(4) Effect Confirmation Test of Branching Trait of Rice Plant of Strigolactone Inhibitor

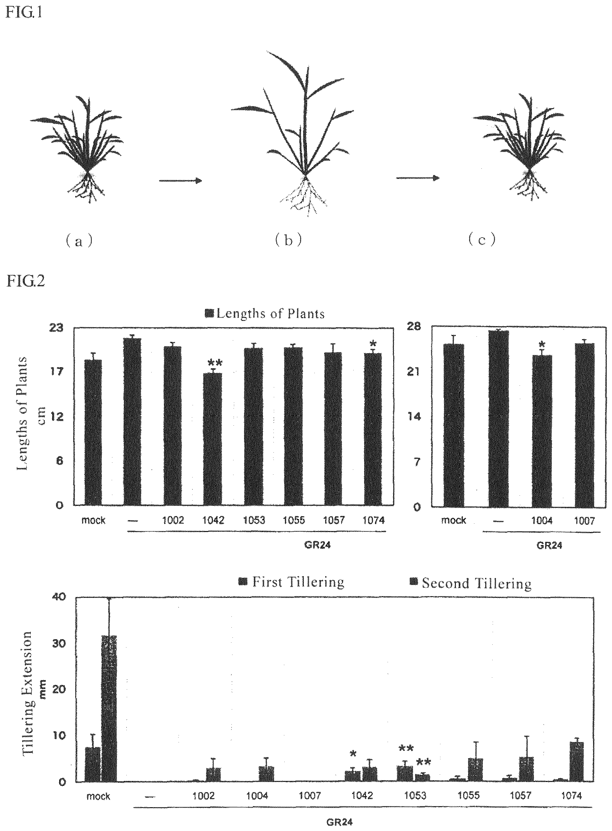

The rice d17 mutant is a mutant strain of a rice plant in which the strigolactone is not endogenous and thus its branching is uncontrollable (FIG. 1 (a)).

When treating the strigolactone analog like GR24 (the following formula (II)) to this d17 mutant, a multiple tillering trait is neutrized to return to the ordinary number of branching (FIG. 1 (b)).

By treating the compounds that indicated the strong strigolactone antagonist activities by the evaluation test in the above-described (3) together with the strigolactone analog GR24 (the following formula (II)), it is expected that the tillering trait does not recover and the multiple tillering remains unchanged (FIG. 1 (c)).

The following test was performed in order to examine whether the compounds synthesized above presented this expected effect or not.

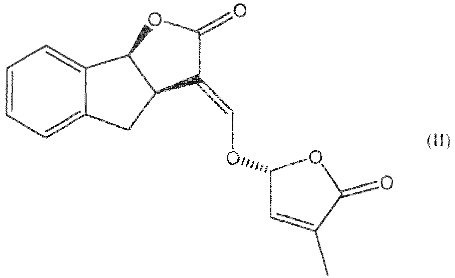

### (5) Evaluation Test (1) of SL Receptor Inhibitor Using Rice d17 Mutant Strain

A sterile solution I (2.5 weight% sodium hypochlorite aqueous solution, 0.01% Tween-20) was applied to rice plant seeds (Shiokari), and the rice plant seeds (Shiokari) were shaken for 15 minutes at a room temperature. The sterile solution I was thrown away, a sterile solution II (2.5 weight% sodium hypochlorite aqueous solution) was applied to the rice plant seeds (Shiokari), and the rice plant seeds (Shiokari) were shaken for 15 minutes at a room temperature (25°C). The sterile solution II was thrown away in a clean bench, the seeds were cleaned with a sterile water for five times, and left to stand in a dark place for two days at 25°C. After two days, the germinated seeds were transplanted to an agar medium for rice plant hydroponics, and was left to stand for six days at 25°C with 16 hours of light period and 8 hours of dark period. A 12 mL of rice plant hydroponics medium containing the compound was added to a 12 mL brown vial bottle, and the rice plant was transplanted from the agar medium and was left to stand for seven days at 25°C with 16 hours of light period and 8 hours of dark period. In a halfway on the fourth day, the rice plant hydroponics medium was replenished 5 mL each.

After leaving to stand for seven days, a first tillering and a second tillering, and lengths of the plants were measured.

FIG. 2 shows the results. The vertical axis indicates each of the lengths of the plants or the tillerings, and the horizontal axis indicates kinds of the administered compounds. Note that, in FIG. 2, 1002 corresponds to the compound 1, 1004 corresponds to the compound 3, 1007 corresponds to the compound 5, 1042 corresponds to the compound 6, 1053 corresponds to the compound 10, 1055 corresponds to the compound 12, 1057 corresponds to a compound 13, and 1074 corresponds to the compound 19.

The GR24 treatment was 1 µM, and 5 µM of the triazole urea compound was treated. Recoveries of tillering extensions were observed, except for the compound 5.

### (6) Evaluation Test (2) of SL Receptor Inhibitor Using Rice d17 Mutant Strain

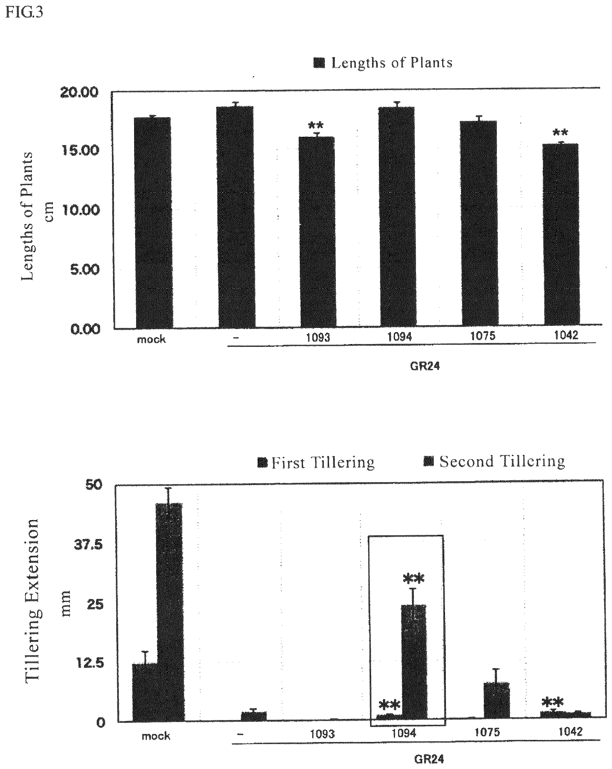

The test similar to that in FIG. 2 was performed for other compounds. The results are shown in FIG. 3. Similar to FIG. 2, the vertical axis indicates each of the lengths of the plants or the tillering, and the horizontal axis indicates the kinds of the administered compounds. In FIG. 3, 1042 corresponds to the compound 6, 1075 corresponds to the compound 20, 1093 corresponds to the compound 24, and 1094 corresponds to the compound 25. The GR24 treatment was 0.1 µM, and 0.5 µM of the triazole urea compound was treated. The compound 25 that was designed by modifying the compound 5, with which the recovery of the tillering extension could not observed in the test in FIG. 2, had the best recovery in the tillering extension among the used compounds.

### (7) Tillering Acceleration Effect Confirmation Test of Wild-Type Rice Nipponbare by Compound 25 Treatment

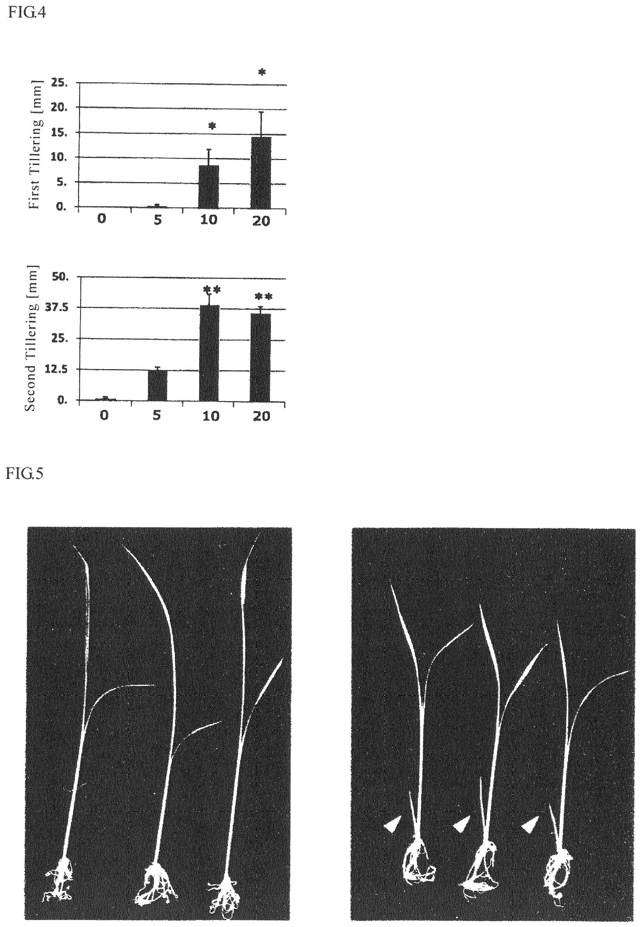

The wild-type rice (variety: Nipponbare) was grown in the hydroponic solution similarly to the tests in FIG. 2 and FIG. 3, on the seventh day from the germination, 10 µM of the compound 25 was added, and the wild-type rice (variety: Nipponbare) was grown for another seven days. The tillering acceleration by the compound 25 treatment was observed. The results are shown in FIG. 4. The vertical axis indicates the lengths of the tillerings and the horizontal axis indicates the administered treatment concentrations of KOK1094. The growing condition of the wild-type rice after two weeks from the germination with an ordinary hydroponic culture is illustrated in FIG. 5 (left side), and the growing condition of the wild-type rice grown in a hydroponic solution added with 10 µM of the compound 25 is illustrated in FIG. 5 (right side).

### (9) Germination Inhibition Test of Striga Seeds

The coadministration of the GR24 or the GR24 and the compound 25 was performed on seeds of *Striga* (*Striga hermonthica*) pretreated for one week at 30°C. The GR24 treatment was performed with 0.1 µM, and the compound 25 treatment was performed with 50 µM or 100 µM. The second table shows the results.

**Second table**

| No. | Concentration of compound 25 (µM) | Concentration of GR24 (µM) | Germination rate of striga seed (%) |
|---|---|---|---|
| 1 | 0 | 0 | 3.17 |
| 3 | 0 | 0.1 | 30.92 |
| 4 | 50 | 0.1 | 2.03 |
| 5 | 100 | 0.1 | 3.13 |

From the second table, it can be found that the compound 25 presents an excellent germination inhibitory effect for *Striga* seeds. When 0.1 µM of the GR24, which is the agonist of the strigolactone, is added, the germination of the *Striga* seeds is accelerated (No. 2), however, coadministering the compound 25 ensures obtaining the germination inhibitory effect of *Striga* seeds (Nos. 3, 4).

## Claims

1. A strigolactone receptor inhibitor for plants, the strigolactone receptor inhibitor containing at least one kind of triazole urea compounds represented by the following formula (I) as an active ingredient. 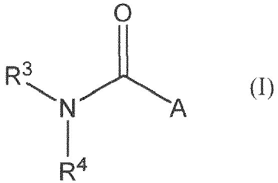 [In the formula, A represents a group represented by the following (A-1) or (A-2). 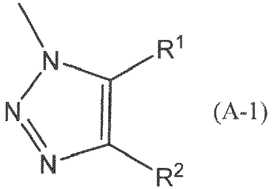 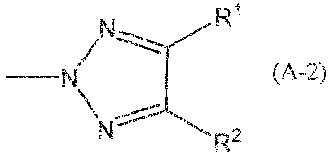 (In the formula, each of R¹ and R² independently represents a hydrogen atom, an alkyl group having 1 to 20 carbon atoms that may have a substituent, or an aryl group having 6 to 20 carbon atoms that may have a substituent.)
Each of R³ and R⁴ independently represents a hydrogen atom, an alkyl group having 1 to 20 carbon atoms that may have a substituent, a cycloalkyl group having 3 to 20 carbon atoms that may have a substituent, or an aryl group having 6 to 20 carbon atoms that may have a substituent (where a case where both R³ and R⁴ are hydrogen atoms is excluded). R³ and R⁴ may form a ring together, and the ring may have a substituent at any position.]

2. The strigolactone receptor inhibitor for plants according to claim 1, wherein
a group represented by -N(R³)(R⁴) in the formula (I) is any one of groups represented by the following (B-1) to (B-7). 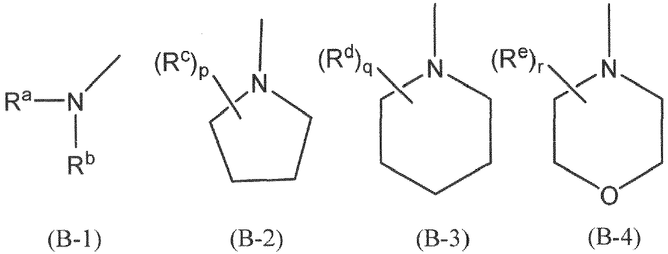 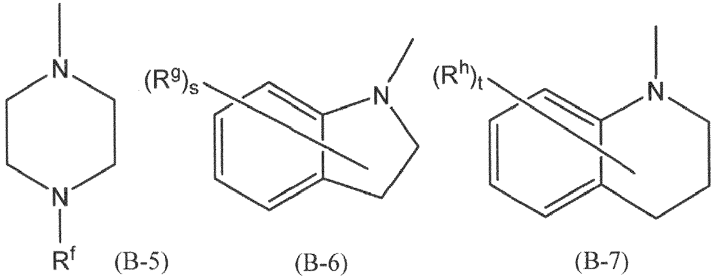 (In the formula, each of R^{a} and R^{b} independently represents an alkyl group having 1 to 20 carbon atoms that may have a substituent or an aryl group having 6 to 20 carbon atoms that may have a substituent.
Each of R^{c}, R^{d}, R^{e}, R^{g}, and R^{h} independently represents a nitro group, a cyano group, a halogen atom, an alkyl group having 1 to 6 carbon atoms, a haloalkyl group having 1 to 6 carbon atoms, a cycloalkyl group having 3 to 20 carbon atoms, an alkoxy group having 1 to 6 carbon atoms, an amino group, a mono-substituted amino group, a disubstituted amino group, an alkylthio group having 1 to 6 carbon atoms, an alkylsulfenyl group having 1 to 6 carbon atoms, an alkylsulfonyl group having 1 to 6 carbon atoms, an alkoxycarbonyl group having 2 to 10 carbon atoms, an alkylcarbonyl group having 2 to 10 carbon atoms, or an aryl group that may have a substituent.
R^{f} represents a hydrogen atom, an alkyl group having 1 to 20 carbon atoms that may have a substituent, a cycloalkyl group having 3 to 20 carbon atoms that may have a substituent, an aryl group having 6 to 20 carbon atoms that may have a substituent, a heterocyclic group that may have a substituent, or an acyl group having 2 to 20 carbon atoms.
Each of *p*, *q, r, s,* and *t* independently represents an integer of 0 to 4.)

3. An agricultural composition that contains at least one kind of triazole urea compounds represented by the following formula (I) and an agriculturally acceptable formulation adjuvant. 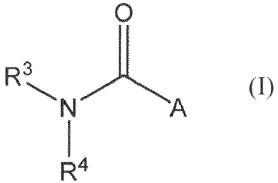 [In the formula, A represents a group represented by the following (A-1) or (A-2). 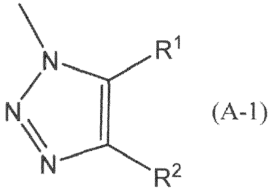 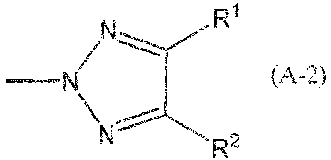 (In the formula, each of R¹ and R² independently represents a hydrogen atom, an alkyl group having 1 to 20 carbon atoms that may have a substituent, or an aryl group having 6 to 20 carbon atoms that may have a substituent.)
Each of R³ and R⁴ independently represents a hydrogen atom, an alkyl group having 1 to 20 carbon atoms that may have a substituent, a cycloalkyl group having 3 to 20 carbon atoms that may have a substituent, or an aryl group having 6 to 20 carbon atoms that may have a substituent (where a case where both R³ and R⁴ are hydrogen atoms is excluded). R³ and R⁴ may form a ring together, and the ring may have a substituent at any position.]

4. The agricultural composition according to claim 3, wherein
a group represented by -N(R³)(R⁴) in the formula (I) is any one of groups represented by the following (B-1) to (B-7). 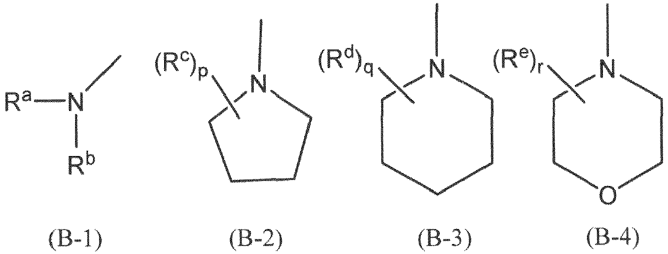 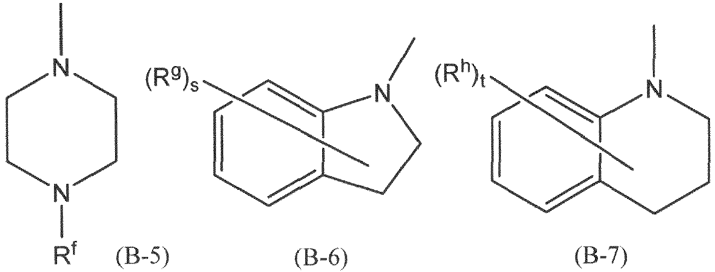 (In the formula, each of R^{a} and R^{b} independently represents an alkyl group having 1 to 20 carbon atoms that may have a substituent or an aryl group having 6 to 20 carbon atoms that may have a substituent.
Each of R^{c}, R^{d}, R^{e}, R^{g}, and R^{h} independently represents a nitro group, a cyano group, a halogen atom, an alkyl group having 1 to 6 carbon atoms, a haloalkyl group having 1 to 6 carbon atoms, a cycloalkyl group having 3 to 20 carbon atoms, an alkoxy group having 1 to 6 carbon atoms, an amino group, a mono-substituted amino group, a disubstituted amino group, an alkylthio group having 1 to 6 carbon atoms, an alkylsulfenyl group having 1 to 6 carbon atoms, an alkylsulfonyl group having 1 to 6 carbon atoms, an alkoxycarbonyl group having 2 to 10 carbon atoms, an alkylcarbonyl group having 2 to 10 carbon atoms, or an aryl group having 6 to 20 carbon atoms that may have a substituent.
R^{f} represents a hydrogen atom, an alkyl group having 1 to 20 carbon atoms that may have a substituent, a cycloalkyl group having 3 to 20 carbon atoms that may have a substituent, an aryl group having 6 to 20 carbon atoms that may have a substituent, a heterocyclic group that may have a substituent, or an acyl group having 2 to 20 carbon atoms.
Each of *p*, *q, r, s,* and *t* independently represents an integer of 0 to 4.)

5. A method for using the strigolactone receptor inhibitor according to claim 1 or 2, or the agricultural composition according to claim 3 or 4 as a branching enhancer for plants.

6. A method for using the strigolactone receptor inhibitor according to claim 1 or 2, or the agricultural composition according to claim 3 or 4 as a tillering accelerator for gramineous plants.

7. A germination inhibitor for *Striga* seeds, the germination inhibitor containing at least one kind of triazole urea compounds represented by the following formula (I) as an active ingredient. 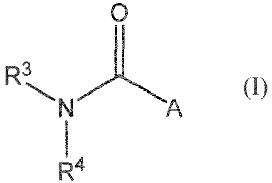 [In the formula, A represents a group represented by the following (A-1) or (A-2). 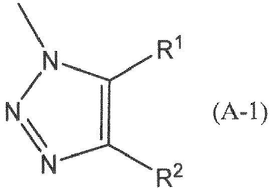 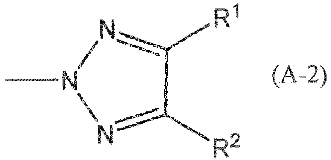 (In the formula, each of R¹ and R² independently represents a hydrogen atom, an alkyl group having 1 to 20 carbon atoms that may have a substituent, or an aryl group having 6 to 20 carbon atoms that may have a substituent.)
Each of R³ and R⁴ independently represents a hydrogen atom, an alkyl group having 1 to 20 carbon atoms that may have a substituent, a cycloalkyl group having 3 to 20 carbon atoms that may have a substituent, or an aryl group having 6 to 20 carbon atoms that may have a substituent (where a case where both R³ and R⁴ are hydrogen atoms is excluded.). R³ and R⁴ may form a ring together, and the ring may have a substituent at any position.]

8. A triazole urea compound represented by the following formula (I-1). 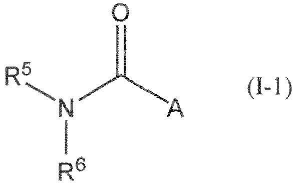 [In the formula, A represents a group represented by the following (A¹-1) or (A¹-2). 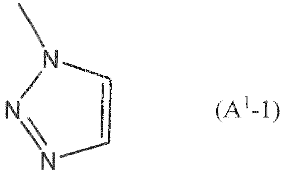 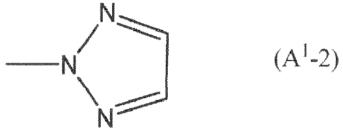 A group represented by -N(R⁵)(R⁶) represents a group represented by the following (B¹-5), (B¹-6), or (B¹-7). 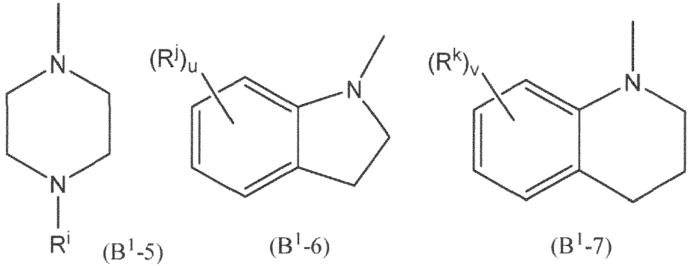 (In the formula, R¹ represents a hydrogen atom, an alkyl group having 1 to 20 carbon atoms that may have a substituent, a cycloalkyl group having 3 to 20 carbon atoms that may have a substituent, an aryl group having 6 to 20 carbon atoms that may have a substituent, or a heterocyclic group that may have a substituent.
Each of R^{j} and R^{k} independently represents a nitro group, a cyano group, a halogen atom, an alkyl group having 1 to 6 carbon atoms, a haloalkyl group having 1 to 6 carbon atoms, a cycloalkyl group having 3 to 20 carbon atoms, an alkoxy group having 1 to 6 carbon atoms, an amino group, a mono-substituted amino group, a disubstituted amino group, an alkylthio group having 1 to 6 carbon atoms, an alkylsulfenyl group having 1 to 6 carbon atoms, an alkylsulfonyl group having 1 to 6 carbon atoms, an alkoxycarbonyl group having 2 to 10 carbon atoms, an alkylcarbonyl group having 2 to 10 carbon atoms, or an aryl group having 6 to 20 carbon atoms that may have a substituent.
Each of *u* and *v* independently represents an integer of 0 to 4.)
